# EUROPEAN PATENT SPECIFICATION

(11) **EP 2 579 916 B1**
(45) Date of publication and mention of the grant of the patent: **06.01.2016**
(21) Application number: 11728584.1
(22) Date of filing: 13.06.2011
(51) Int. Cl.: A61M 5/14, A61M 5/142, A61M 5/00, A61M 5/145

(54) **APPARATUS AND CASE FOR INFUSION EQUIPMENT**
VORRICHTUNG UND GEHÄUSE FÜR EIN INFUSIONSGERÄT
APPAREIL ET BOÎTIER POUR ÉQUIPEMENT DE PERFUSION

(30) Priority: 11.06.2010 GB 201009812
(43) Date of publication of application: 17.04.2013
(73) Proprietor: Britannia Pharmaceuticals Limited, Newbury, Berkshire RG14 1JN (GB)
(72) Inventor: NOWAK, Rachael, Newbury, Berkshire, RG14 1JN (GB); SHAW, Andrew, Coventry, Warkwickshire, CV8 3HG (GB); MARTIN, Graham, John, Coventry, Warkwickshire, CV8 3HG (GB)
(74) Representative: Kernebeck, Thomas
(86) International application number: PCT/EP2011/002897
(87) International publication number: WO 2011/154160

(56) References cited:
- EP-A2- 0 354 324
- WO-A2-2009/098648
- US-A1- 2007 073 228
- US-A1- 2009 275 887

## Description

The present invention relates to an apparatus for infusion equipment, including a portable case for holding a vessel having a tubular bore and an actuator pump for the vessel, kits comprising a case, a pump and a vessel having a tubular bore and corresponding methods.

It is known in the art to provide a pre-filled vessel such as an ampoule or syringe for holding a liquid, usually a drug for medical use. In view of the nature of some drugs, such ampoules often need to be made of a brittle material such as glass. Although an ampoule made of a more resilient material such as some plastic materials would be more practical, the nature of certain drugs means they cannot be retained in plastic for any length of time because the drug is not stable held in plastic in the long-term. There are many reasons for this. One reason is that plastics are gas permeable and thereby allow oxygen to enter the ampoule and cause oxidation. One example of a drug which would be adversely affected in this way is apomorphine, used to treat Parkinson's disease. Another reason is that plastics have certain leachables and extractables that can be released into aqueous solutions and destabilize the drug or one of its excipients. For these and other reasons such drugs must be supplied to a user in a glass ampoule or vial.

Such drugs are often used together with an infusion pump. When the pump and a syringe are attached together, a plunger of the pump pushes a piston of the syringe to deliver the drug into the patient. For many drugs the infusion occurs over a period of time and thus the pump and syringe must be carried on the person of the patient. The drug is administered from the syringe via a tube and canula.

WO 2009/098648 A2 discloses a drug delivery device in which a disposable unit has a channel which accomodates a cartridge containing the drug to be delivered and a loading spring for applying a loading force on a piston of said cartridge.

At present there is no means of using a glass syringe directly with an infusion pump. Thus in order to set the drug infusing, the patient must first decant the drug from the glass ampoule into a plastic syringe that is part of the delivery system. The plastic syringe is attached to the infusion pump.

One example of a drug administered by this system is a drug used to treat Parkinson's disease. This is a degenerative disease that causes, among other things, severe shaking of the body and consequent difficulty in controlling the limbs and associated loss of dexterity. Thus it can be understood that one problem with the known system of administration is the requirement to decant a drug from a glass ampoule to a plastic syringe. Even if a carer or healthcare professional were to perform the decanting on behalf of a patient there is an inherent risk of spillage of the drug which would thereby leave an incorrect dosage for infusion into the patient. If a patient with a disease such as Parkinson's needs to perform the decanting themselves this presents considerable difficulties because of the need for a steady hand. Parkinson's attacks can be unpredictable and thus can strike at any time, which means a patient might well be on their own when the drug needs to be replenished. Furthermore, if delivered in an ampoule the drug may need to be diluted before use, which is another potential source of inaccuracy in the dosage given to the patient. The transfer of drug from the ampoule to the syringe might also increase the risk of contamination of the drug.

Another problem with prior art systems is that there is no convenient way for a patient to carry the syringe and pump around with them in a secure and comfortable manner. Also, the syringe and pump are liable to physical damage from for example water, light, knocks, dirt etc. as they are likely to be unprotected on the patient. Damage to the syringe or ampoule can result in leakage of the drug product, which in the case of some drugs such as apomorphine used to treat Parkinson's can be a skin irritant. Furthermore, leaked drug can oxidize to a coloured product which can stain the skin, clothing and other materials that it leaks onto. Typically, pumps are not water resistant and this is a problem when it rains or comes into contact with water on a daily basis.

It would be desirable to mitigate the above-mentioned problems.

The invention is set out in claims 1 and 13-15.

According to a first aspect of the present invention, there is provided an apparatus for infusion equipment comprising a vessel having a tubular bore and a separate actuator pump for the vessel, said apparatus comprising : a holder shaped and dimensioned to hold the vessel; and a pump attachment with which the holder can be engaged, the attachment being shaped and dimensioned to be removeably engagable with an actuator pump, to thereby, when the holder and pump attachment are so engaged, removeably hold the pump and vessel in mutual fixed relation such that the actuator pump is operably engaged with a piston of the vessel without attaching the vessel to the pump.

The arrangement of the apparatus is such that when a vessel is held by the holder and the holder is engaged with the pump attachment, movement of the vessel in a direction in which a plunger of the pump pushes a piston of the vessel is prevented or substantially prevented.

In some embodiments the pump attachment and the holder comprise corresponding engageable means for allowing the said engagement. In some embodiments the engageable means can be engaged for securely fitting the holder to the pump attachment. The engageable means could take the form of corresponding screw threads or other mechanically interacting elements such as a detent mechanism or by close fitting.

In many cases the pump attachment is shaped and dimensioned to be removeably engageable with an actuator pump by means of one or more wings on one of the pump attachment and the pump and corresponding one or more recesses on the other of the pump attachment and the pump which wings and recesses interengage. If appropriate for a particular pump, the wings can comprise a stepped radius arranged to engage with a corresponding stepped recess on the pump.

Suitably, when engaged, the holder and the pump attachment define a tubular bore into which a vessel can fit snugly. Preferably the tubular bore is arranged to cover a substantial part of the vessel.

Preferably a substantial part of the tubular bore is formed by the holder.

Advantageously, when engaged, the holder and the pump attachment are arranged to limit movement of a vessel out of the bore towards an end distal from the pump attachment. Such movement limitation can be effected by tapering of the bore.

Conveniently, the movement of a vessel is limited such that, when a vessel is held in the holder, an open end of the vessel is enveloped by the bore to thereby protect it.

Preferably the holder comprises user-operable means for assisting engagement with and disengagement from the pump attachment.

In preferred embodiments, the apparatus further comprises a case defining an internal cavity that is shaped and dimensioned to accommodate the holder and pump attachment when fitted together and holding a vessel removeably engaged with a pump. Advantageously the case is shaped and dimensioned to cover a substantial proportion of the holder and pump attachment.

Suitably, the case comprises two halves, and a first half comprises the pump attachment, which first half is arranged to accommodate the holder. Preferably the other half of the case is attachable to the first half of the case and is for accommodating a pump.

Advantageously the case is arranged to allow a user-operable means on the holder to project from the case for insertion and removal of the holder into the case.

According to second aspect of the present invention, there is provided a kit comprising : a pre-filled vessel having a tubular bore; an actuator pump for the pre-filled vessel; a holder for holding the vessel; and a pump attachment with which the holder can engage, the attachment being shaped and dimensioned to be removeably engagable with the actuator pump, to thereby, when the holder is engaged with the pump attachment, removeably hold the pump and vessel in mutual fixed relation such that the actuator pump is operably engaged with a piston of the vessel without attaching the vessel to the pump.

Advantageously the kit further comprises a case for accommodating the holder, the pump and the pump attachment.

According to a third aspect of the present invention, there is provided a method of holding infusion equipment, comprising a vessel having a tubular bore and a separate actuator pump for the vessel, comprising : inserting a pre-filled vessel into a holder; engaging the holder with a pump attachment; and removeably engaging the pump attachment with an actuator pump to thereby hold the pump and vessel in mutual fixed relation such that the actuator pump is operably engaged with a piston of the vessel without attaching the vessel to the pump.

Advantageously the method further comprises inserting the holder, pump attachment and actuator pump into a case.

According to a fourth aspect of the present invention, there is provided a portable case for infusion equipment comprising a vessel having a tubular bore and a separate actuator pump for the vessel, said case having a shell-like body defining an internal cavity that is shaped and dimensioned to accommodate said pump and vessel, said case being shaped within the cavity for removably holding the pump and vessel in mutual fixed relation such that the actuator pump is operably engaged with a piston of the vessel without attaching the vessel to the pump.

Preferably the case is shaped completely to envelop the vessel and pump. Suitably the case comprises two halves and means for releasably securing the two halves together in a closed arrangement, thereby to enclose the pump and vessel. The two halves may be hinged together for opening and closing the case.

Advantageously the case is configured within the cavity to support stably the pump and vessel around their entire extents. Preferably the case comprises, within the cavity, a plurality of formations for supporting the pump and vessel. The formations may define a first recess that is configured to receive snugly the vessel and a second recess that is configured to receive snugly the pump.
They may comprise locating means arranged to facilitate location of the pump and/or vessel in the first and/or second recess and to engage with the pump and/or vessel to facilitate the said holding in mutual fixed relation. Conveniently the locating means comprises a plurality of ribs.

Advantageously the internal cavity comprises one or more finger recesses or releasable tabs to facilitate insertion into and removal of the pump and/or vessel from the case.

The means for releasably securing the two halves together may comprise a fastening means, the fastening means being operable to securely close the case or allow the case to be opened. Suitably the fastening means comprises a buffer means and a user-operable means. The user-operable means may comprise a textured portion which can be felt by a user to assist in opening and closing the case.

Preferably the shell-like body is substantially rigid and comprises flexible, stiffening formations.

The apparatus or case can further comprise an outlet port for exiting a cannula connected to the vessel from the case. The apparatus or case advantageously comprises a seal which acts between the two halves when secured together.

The vessel of the infusion equipment can comprise a cylindrical barrel defining the tubular bore along which the piston can move. The vessel may be a syringe; a cartridge; or a carpoule.

According to a fifth aspect of the invention, there is provided a kit comprising : a portable case; a pre-filled vessel having a tubular bore and able to be held within the case; and an actuator pump for the pre-filled vessel able to be held within the case, wherein the case is arranged for holding the pump in mutual fixed relation with the vessel such that the pump is operably engaged with a piston of the vessel.

Preferably the vessel of either of the aforementioned kits has a flangeless end at which it can be pre-filled with a liquid, and advantageously the flangeless end is arranged to be in unattached contact with the pump when the pump and vessel are operably engaged.

The vessel of either of the aforementioned kits may comprise a brittle material and the case may be arranged to hold the vessel in mutual fixed relation with the pump such that the vessel is substantially protected from shattering.

The vessel of the infusion equipment can comprise a cylindrical barrel defining the tubular bore along which the piston can move. The vessel may be a syringe; a cartridge; or a carpoule.

According to a sixth aspect of the invention, there is provided a method of holding infusion equipment comprising a vessel having a tubular bore and a separate actuator pump for the vessel, comprising inserting a pump and a pre-filled vessel in a portable case, said case having a shell-like body defining an internal cavity that is shaped and dimensioned to accommodate said pump and vessel, such that the pump and vessel are removably held in mutual fixed relation and the actuator pump is operably engaged with a piston of the vessel without attaching the vessel to the pump.

Embodiments of the invention will now be described, by way of example only, with reference to the accompanying drawings in which :
Figures 1a-c show three three-dimensional views of a first embodiment of a case in accordance with the invention, in an open configuration;
Figures 2a&b show two three-dimensional views of the case of figure 1 in a semi-open configuration;
Figures 3a-e show five views of the case of figure 1 in a closed configuration;
Figures 4a-c show three three-dimensional views of the case of figure 1 with a pump device and syringe device held therein, in an open configuration;
Figure 5 shows details of the structure of the case of figure 1 which holds a syringe device and a pump device;
Figure 6 shows details of the structure of the case of figure 1 viewed from a different angle;
Figures 7a and 7b show two prior art methods of preparing a syringe of medicine;
Figure 8 shows the prior art syringe of figures 7a and 7b attached to an infusion pump;
Figure 9 shows a top view of a case according to a second embodiment of the invention;
Figure 10 shows an underside view of the case of figure 9;
Figure 11 shows an end view of the case of figure 9, shown from the end in which a syringe holder is accommodated;
Figure 12 shows a view of the other end of the case of figure 9, shown from the end in which a pump isaccommodated;
Figure 13 shows a side view of the case of figure 9;
Figures 14a and b show perspective views of the case of figure 9, on the top and underside respectively;
Figure 15 shows a cross-section through the case of figure 9 when holding a pump and syringe.
Figure 16 shows a syringe with cannula which can be accommodated in the case of figure 9;
Figure 17 shows a syringe in a syringe holder in position to be inserted into the case of figure 9;
Figure 18 shows a syringe held by the syringe holder and pump attachment which form part of the case of figure 9, together with a pump; and
Figure 19a shows a front half of the case of figure 9 with a pump in position to be engaged with a syringe in a syringe holder and figure 19b shows the pump so engaged and the rear half of the case in position to be attached to the front half. In the figures, like reference numerals indicate like parts.

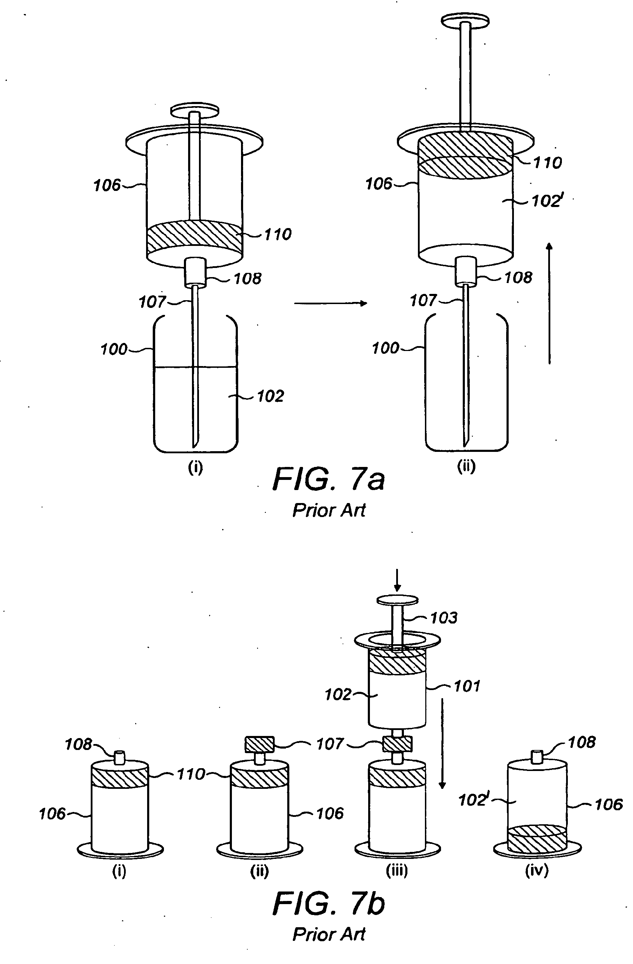

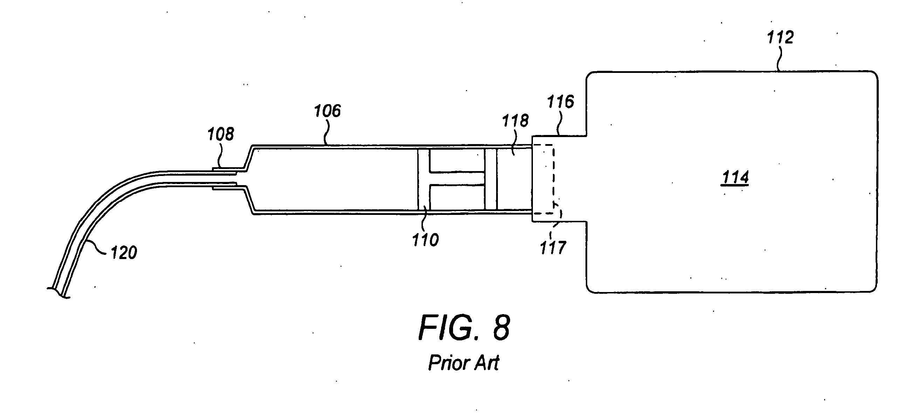

Referring firstly to the prior art figures 7 and 8, figure 7a shows a first prior art method for obtaining a plastic syringe of medicine for use with a pump. The arrangement comprises a glass ampoule 100 filled with a liquid medicine 102. In this case the liquid medicine 102 is for treating Parkinson's disease but the arrangement shown is used for other drugs. The first step to be carried out by the user is to break the seal of the ampoule 100 (not shown). Secondly, a plastic syringe 106 is used to draw the liquid medicine 102 up into the plastic syringe 106 using a needle 107 (which must be fitted to a neck 108 of the plastic syringe 106). The arrangement at this stage is shown in picture (i). A piston 110 moves up the syringe 106 to allow the liquid medicine 102 to be accommodated. A third step is to draw up the same volume of a diluent (usually purified water or saline solution). Picture (ii) shows the syringe 106 after the second and third steps. The diluted medicine is indicated with reference numeral 102'. After this, the filled syringe 106 must then be inverted and any air bubbles removed. This is problematic as it can be difficult to remove all the air bubbles and they can be dangerous if the liquid medicine is subsequently administered into a patient's bloodstream. Finally a cannula line is added in place of the needle 107 into the neck 108 of the syringe. The neck 108 is at the end of the syringe now distal from the piston 110.

An alternative prior art method is shown in figure 7b. This method involves use of a pre-filled glass syringe (PFS) 101. The first picture (i) shows the plastic syringe 106. A first step is to attach a connector 107 to the plastic syringe 106 onto the neck 108. The second picture (ii) shows the plastic syringe 106 after this step. The next step (shown in picture (iii)) is to attach the PFS 101 to the connector 107. Then a piston 103 of the PFS 101 is pushed down towards the connector 107 to thereby transfer the (pre-diluted) liquid medicine 102' into the plastic syringe 106. This step is repeated if necessary to transfer all the liquid. Finally, the connector 107 is removed leaving the filled plastic syringe 106 ready to have a cannula line attached. This state is shown in picture (iv).

It will be appreciated that the step of drawing (in the case of figure 7a) or pushing (in the case of figure 7b) the liquid medicine 102,102' from one container to another is liable to result in spillage and is particularly difficult for someone with an unsteady hand, such as a Parkinson's disease sufferer. The liquid medicine 102 is provided in a precise dosage so loss of any of the liquid 102 would result in an incorrect amount being administered to the patient. Furthermore, the need to either break the ampoule seal and attach a needle or fit a connector and attach the PFS to it can also be difficult, as well as being time-consuming.

The reason that the drawing or pushing step is required is that the medicine 102 is unstable when stored in plastic for a period of time, hence it must be stored and delivered to the patient in a glass ampoule 100 or a PFS 101. However, neither the glass ampoule 100 nor the PFS 101 can be used directly with an actuator pump 112 as shown in figure 8 because glass is too brittle and would be at risk of shattering. This is explained further with reference to figure 8 below.

Figure 8 shows the plastic syringe 106 attached to the actuator pump 112. The actuator pump 112 comprises a main pump body 114 which has a narrower region 116 at one end holding a plunger 118. The pump 112 can be programmed to operate in dependence on a particular application. The plunger 118 moves gradually out of the pump 112 as the pump operates in accordance with the program and thereby pushes the piston 110 down the plastic syringe 106. Thus the liquid 102' is gradually dispensed out of the syringe 110 and into a cannula line 120 fitted in the neck 108 of the syringe 106.

It can be seen in figure 8 that the open end of the syringe 106 (i.e. distal from the neck 108) fits over the plunger 118. In fact the very end of the open end of the syringe 106 slots into a recess 117 (not directly visible in the figure) in the narrower region 116 of the pump 112. Thus the plastic syringe 106 is firmly attached to the pump 112 during operation.

The arrangement of figure 8 could not be used if the plastic syringe 106 were made of glass because the open end of the syringe which fits into the recess 117 would be at risk of shattering during insertion. A glass syringe would also be at risk of shattering once the pump 112 and syringe were set up and dispensing the medicine 102' because the pump 112 and syringe must be carried on the person of the patient. Some drugs, such as those used in the treatment of Parkinson's disease are administered slowly over several hours so carrying round an exposed, brittle syringe would be inconvenient and unsafe for the patient.

Embodiments of the invention will now be described.

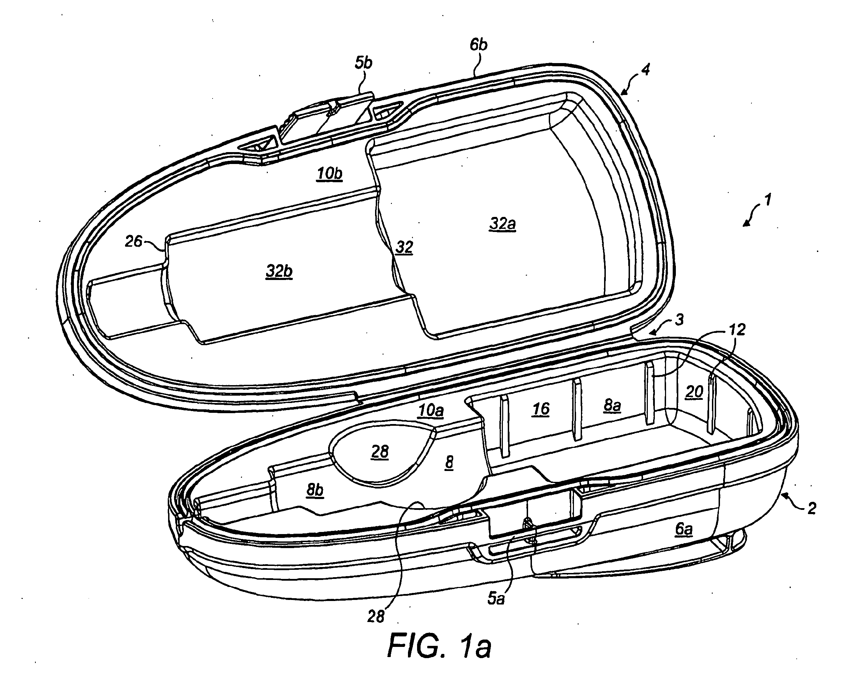

Figures 1a-c show three views from different angles of a portable case 1 in accordance with a first embodiment of the invention. In these views the case 1 is in a substantially open configuration, although it will be appreciated that the actual and maximum degree of opening can vary.

The case 1 has a first portion 2 and a second portion 4. The two portions 2,4 are both of a generally cuboid but rounded shape, having a length greater than a width, the width being greater than a depth. Each is generally hollow and open at one of its largest faces. The lengths and the widths of the open face of each portion 2,4 are sized and shaped to match such that the first 2 and second 4 portions are able to be fitted together at their open faces to form an enclosure. When fitted together, the case has a generally cuboid, rounded shape. The two portions 2,4 are joined along part of a first of each of their longest edges at their open faces by a hinge 3. The hinge 3 extends from near to a first end of the first longest edges to a little over half-way along the first longest edges. At the second end of the first longest edges in the region beyond the extent of the hinge 3 the two portions 2,4 are more rounded in shape than at the first end. The first end of the first longest edges corresponds to a first end of the case 1 and the second end of the first longest edges corresponds to a second end of the case 1.

On the first portion 2 of the case 1 there is a first half 5a of a clasp 5. This first half 5a of the clasp 5 is disposed on a second longest edge of the open face of the first portion 2. Similarly, a second half 5b of the clasp 5 is disposed on a second longest edge of the open face of the second portion 4. Thus the hinge 3 can be rotated to bring the first 2 and second 4 portions together (forming an enclosure as mentioned above), such that the first half 5a and the second half 5b of the clasp 5 are brought together to fasten the case 1 shut. The clasp 5 is releasable for opening the case 1.

It can be seen in the figures that both the portions 2,4 of the case 1 comprise an outer layer 6. This is labeled as 6a on the first portion 2 and 6b on the second portion 4. This outer layer 6 provides the primary structure of the case 1 and is made of a rigid thermoplastic such as acrylonitrile butadiene styrene (ABS). Both portions 2,4 also comprise an inner layer 10, defining the extent of the enclosure formed when the first 2 and second 4 portions are brought together to close the case 1. This inner layer 10 is made of a more flexible plastic such as polypropylene. The inner layer 10 fits snugly in the outer layer 6 with an interference fit. The two are then bonded with adhesive.
Thus the outer layer 6 and the inner layer 10 together form a shell-like body of the case 1 and define an internal cavity.

The inner layer 10a in the first portion 2 of the case 1 and the inner layer 10b in the second portion 4 of the case together provide various formations for holding and supporting a pump and syringe. In the first portion 2 of the case 1, the inner layer 10a forms the main holding means for holding a pump and a syringe. In the second portion 4 of the case 1, the inner layer 10b forms a retaining means for assisting in secure holding of a pump and syringe in the case 1.

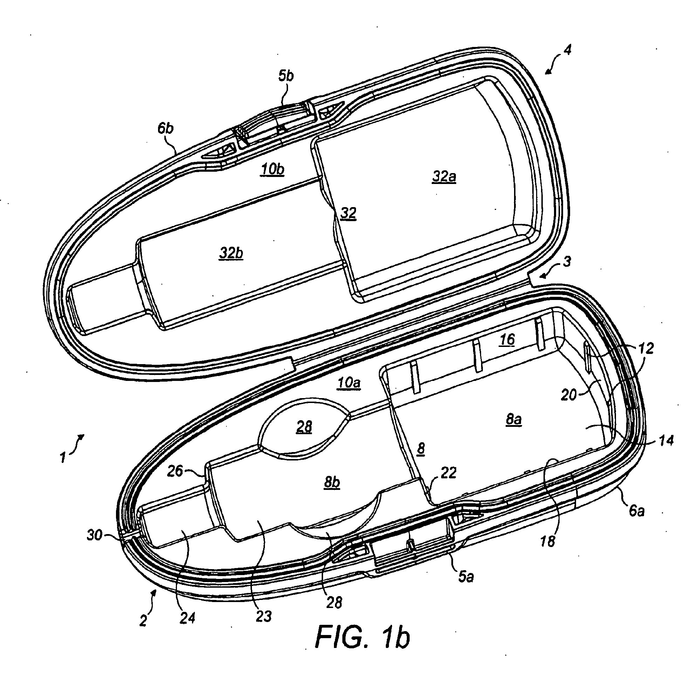

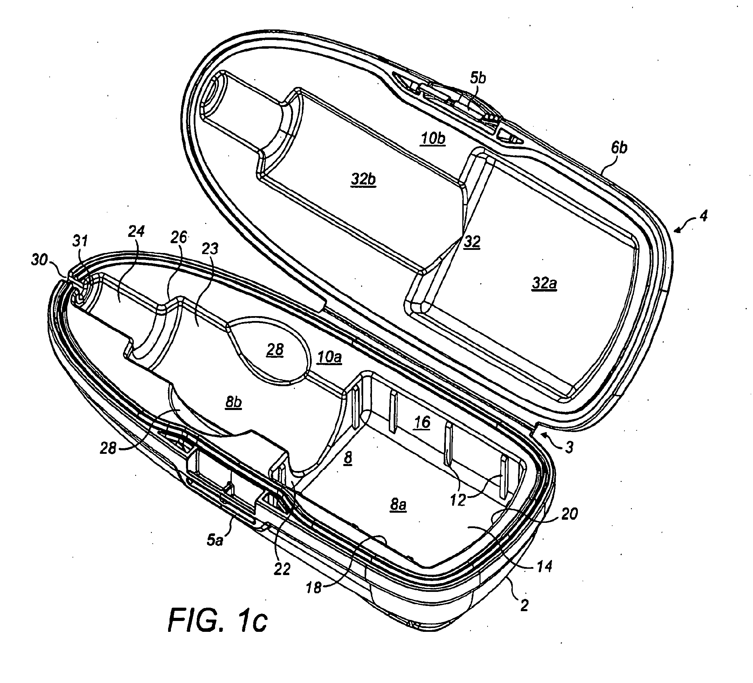

The inner layer 10a of the first portion 2 comprises a shaped recess 8 which can best be seen in figures 1b and 1 c. The shaped recess 8 is formed in two connected sections, a first section 8a and a second section 8b. The recess 8 is able to hold a pump and a syringe as will be shown in subsequent figures. The recess 8 is open to correspond to the open faces of the first 2 and second 4 portions of the case 1.

The first section 8a of the recess 8 is for holding a pump. This first section 8a is located generally towards a first end of the case 1 (as defined previously by the location and extent of the hinge 3), at the right hand end of the case 1 in the figures. The first section 8a is generally rounded cuboid in shape to match the shape of an infusion pump to be held in the case 1. The first section 8a of the recess 8 has a length, width and depth defined to correspond respectively to the length, width and depth of the first portion 2. The first section 8a extends over just less than half of the first portion 2 of the case 1, such that the length of the first section 8a is just less than half the length of the first portion 2 but still longer than the width of the first section 8a. An infusion pump can be inserted into the first section 8a in a direction perpendicular to an open face 14 of the first section 8a. The four other (smaller) faces of the first portion 8a of the recess 8 bear a number of ribs 12 running from top to bottom in the figures, corresponding to a direction of insertion of a pump. The ribs 12 extend down the full depth of the first portion 8a of the recess 8. These ribs 12 act as locators for accurate insertion of a pump and assist in providing a close fit of a pump in the recess 8. Thus they facilitate firm holding of a pump in the recess 8.

In this embodiment there are three evenly-spaced ribs 12 on each of the two longest faces 16, 18 of the four other faces of the first section 8a of the recess 8, two evenly-spaced ribs 12 on a shorter one 20 of the four faces at the first end of the case and two ribs 12 on the other shorter face 22. The two ribs 12 on the other shorter face 22 are disposed close to the longest edges of the first section 8a as can best be seen in figure 1c. This is because this other shorter face 20 leads directly into a second section 8b of the recess 8.

The second section 8b of the recess 8 is for holding a syringe. This second section 8b is generally hemi-cylindrical in shape to correspond to the shape of a syringe. Thus the aforementioned other shorter face 22 of the first section 8a distal from the first end of the case 1 is not a rounded rectangle like the other three smaller faces 16, 18, 20 but instead is a rounded rectangle with a semicircle cut out of it. This semi-circle is upside-down in the direction of insertion of a pump into the first section 8a of the recess 8. Thus it can be understood that a syringe can be inserted into the second section 8b of the recess 8 in the same direction, such that it sits in the hemi-cylindrical shape of the second section 8b.

The depths of the first 8a and second 8b sections of the recess 8 are set such that when a pump and a syringe are respectively inserted into the two sections, when fully inserted to the limit of the depths they can be operably engaged.

The second section 8b of the recess 8 is not a perfect hemi-cylinder but instead comprises a wider, generally hemi-cylindrical region 23 extending from the first section 8a for around three-quarters of the remaining length of the case 1. The end of the second section 8b distal from the first portion 8a forms a region comprising a narrower hemi-cylinder 24. This hemi-cylindrical region 24 is shaped and sized to receive an end of a syringe distal from a pump into which is attached a tubing for delivery of a drug to a patient via a cannula. There is a short connecting region 26 over which the cross-section of the wider region 23 narrows into the cross-section of the narrower region 24.

Another feature of the second section 8b of the recess 8 is that the wider region 23 comprises a finger recess 28 on each side. These are of a rounded shape and are designed to assist in insertion and removal of a syringe from the second section 8b of the recess 8.

As best shown in figure 1c, the very end of the second portion 8a of the recess 8 distal from the first end of the case finishes in a cross-section 31 of a semi-circle with a semi-circle cut out at the top. This smaller semi-circle forms a notch sized to allow a cannula tubing to pass through it. The inner layer 10a and the outer layer 6a both have a corresponding notch for allowing a tubing to pass through from the recess 8 and out of the case 1. These three notches are shown generally by reference numeral 30.

Considering now the second layer 10b of the inner layer 10 disposed in the second portion 4 of the case 1, as previously mentioned this is arranged to facilitate retention of a pump and a syringe in the case 1. Thus this second layer 10b comprises, in a similar manner to the first layer 10a, a shaped recess 32. The shaped recess 32 comprises a first section 32a corresponding to the first section 8a of the recess 8 of the first portion 2 of the case 1. The shaped recess 32 further comprises a second section 32b corresponding to the second section 8b of the recess 8 of the second portion 4 of the case 1. The sections 32a,b of the recess 32 of the second inner layer 10b are generally matched in shape to the sections 8a,b of the recess 8 of the inner layer 10. However, there are no ribs 12 in the first section 32a for retaining a pump. Furthermore there are no finger recesses 28 in the second section 32b because the second section 32b is for retaining a syringe in place but is not intended for insertion and removal of a syringe. There are no corresponding notches in the second section 32b of the recess 32 in the second portion 4 or the inner layer 10b and outer layer 6b of the second portion 4 of the case 1 because a tubing can be entirely incorporated in the notch 30.

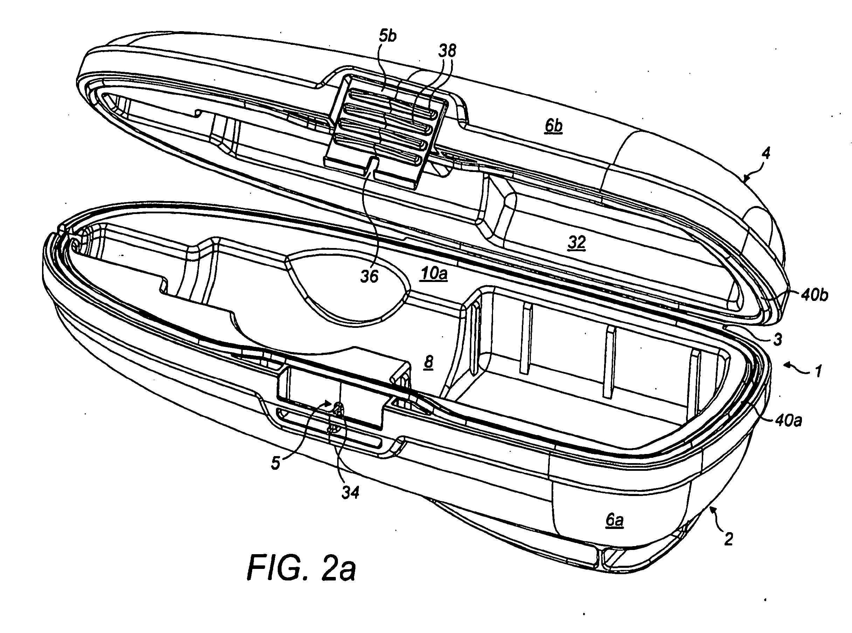

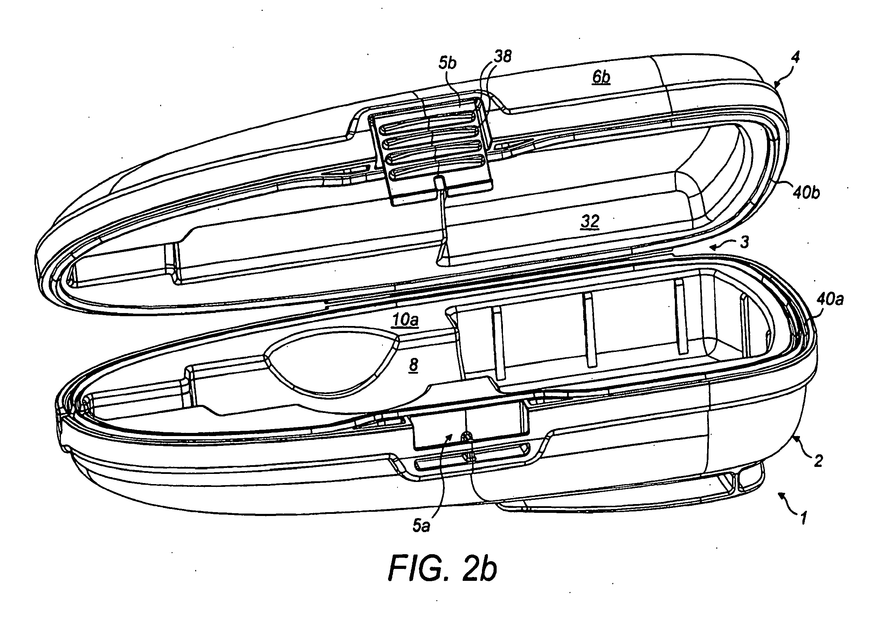

Turning now to figures 2a and 2b, these show two views from different angles of the case 1 in a partially-closed configuration. These figures enable the detail of the clasp 5 to be more clearly seen. The clasp 5 is formed as an integrally-moulded part of the inner layer 10 of the case 1.

It can be seen that the first portion 5a of the clasp 5 on the first portion 2 of the case 1 is formed as a recess in the outer portion 6a of the first portion 2 of the case 1. This recess is for accommodating the second portion 5b of the clasp 5. The first portion 5a of the clasp 5 includes a rubber buffer 34 within the recess. This buffer 34 facilitates correct engagement of the first 5a and second 5b portions of the clasp 5 and mitigates wear of the clasp 5.

The second portion 5b is a generally squareish, planar structure which projects from the second portion 4 of the case 1 in a direction perpendicular to the missing face of the second portion 4a. Thus as the case 1 is closed, the second portion of the clasp 5 moves into the recess of the first portion 5a. The second portion 5b includes a notch 36 which is arranged to co-operate with the rubber buffer 34.

The second portion 5b comprises a number of slightly rounded rib-shaped projections 38 which form a texture to the surface on its outer face. These projections 38 provide an ergonomic means for a user to operate the clasp 5 and in particular provide tactile feedback to assist a user with poor vision in orientating the case and locating the second portion 5b of the clasp to fasten or release the clasp 5.

Figures 2a and 2b also show a view of a rubber seal 40 which provides a seal between the two halves 2, 4 of the case 1 when it is closed. The rubber seal 40 comprises a first part 40a disposed on the first portion 2 of the case 1 and a second part 40b disposed on the second portion 4 of the case 1. The first part 40a comprises a recess running around the open face of the first portion 2 and the second part 40b comprises a ridge running around the open face of the second portion 4. Thus when the case 1 is closed the ridge 40b slots into the recess 40a. In view of the elastic nature of rubber, the interaction of the ridge 40a and the slot 40b seals the case 1 to assist with maintaining it closed. This seal improves water resistance and thereby protection for the contents of the case from dirt, dust, light, water and saltwater etc.

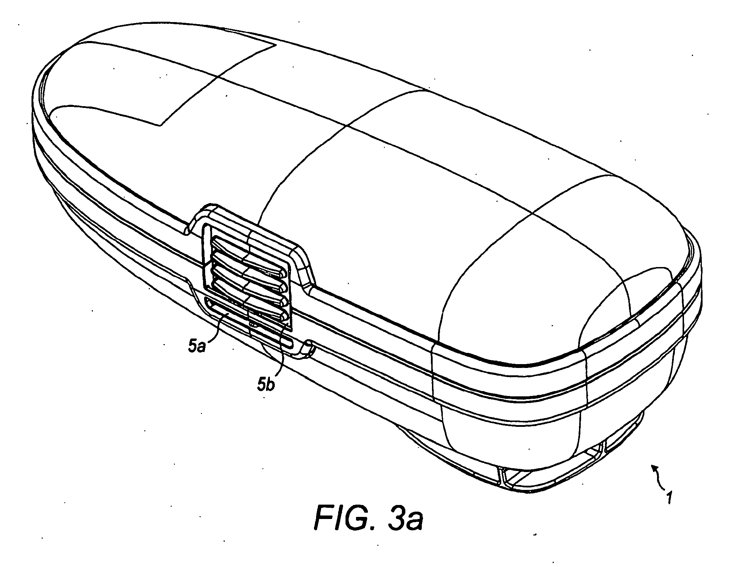

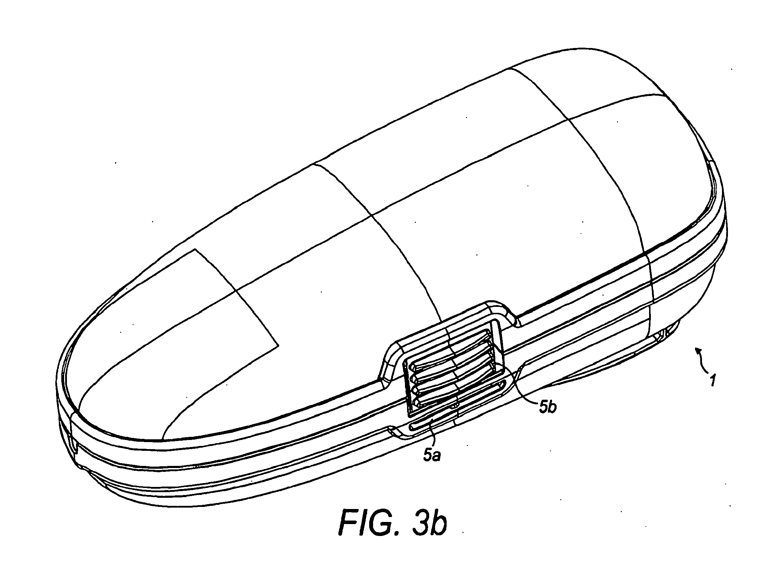

Figures 3a and 3b show the case 1 of figures 1 and 2 when fully closed. They are both three-dimensional views but taken from different angles. The first portion 5a of the clasp 5 is engaged with the second portion 5b of the clasp 5.

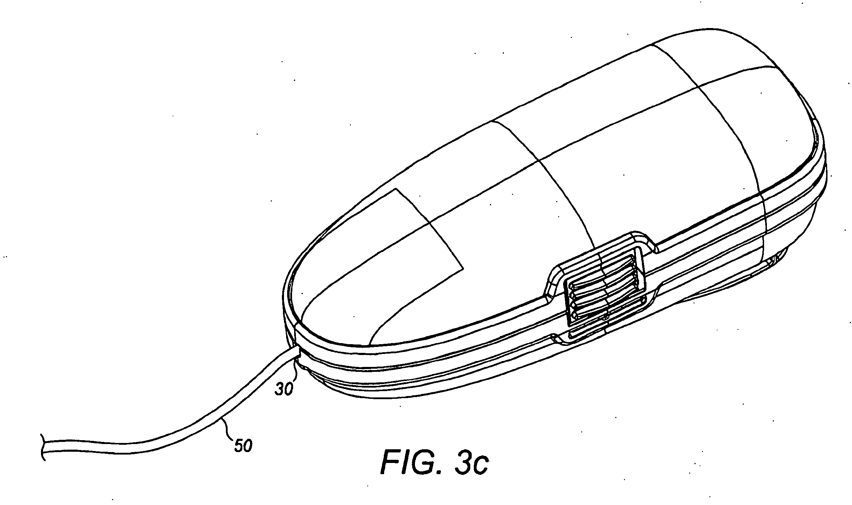

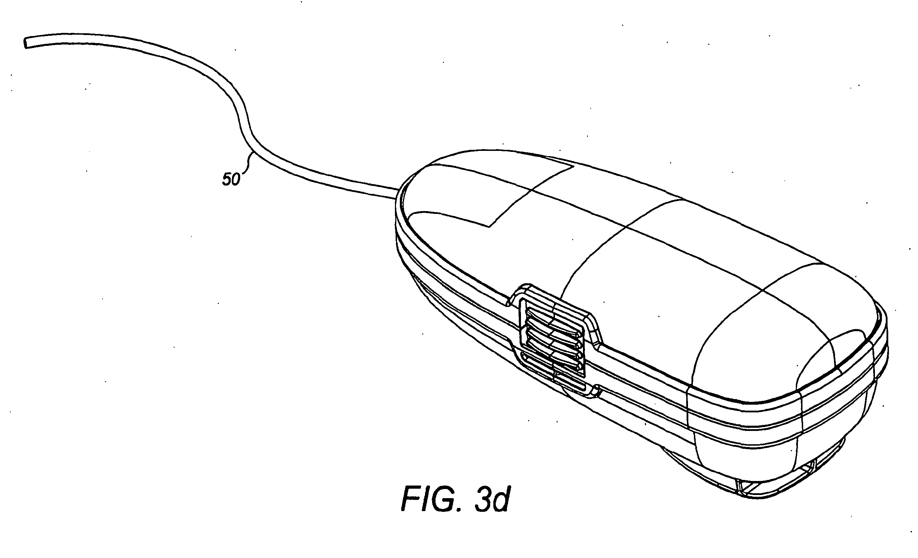

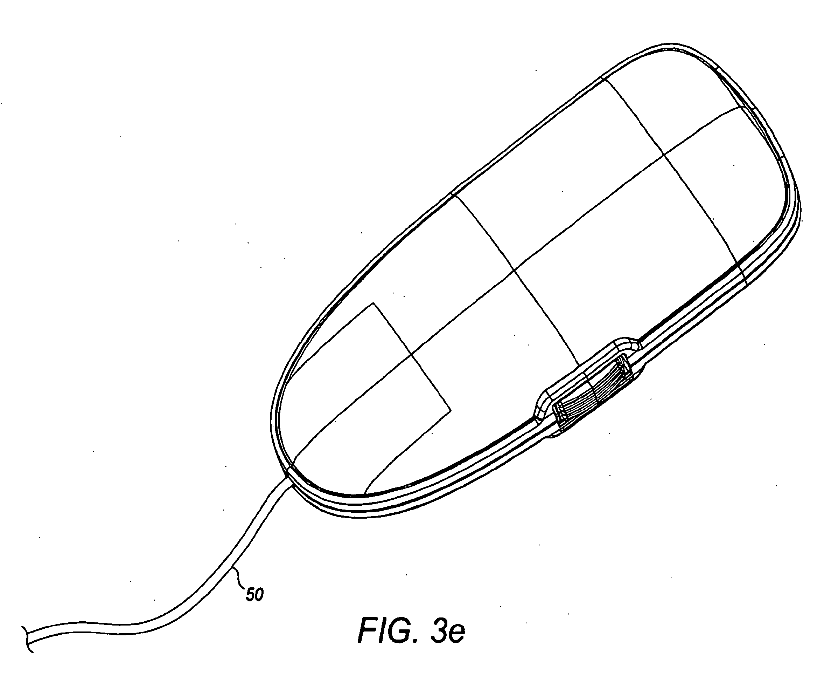

Figures 3c and 3d show two further three-dimensional views of the case 1 when fully closed and figure 3e shows a top view. In figures 3c-e a cannula line 50 is shown exiting the case 1 at the notch 30 (the notch 30 is only visible in figure 3c). The cannula line 50 is attached to a syringe when a pump and syringe are held in the case 1.

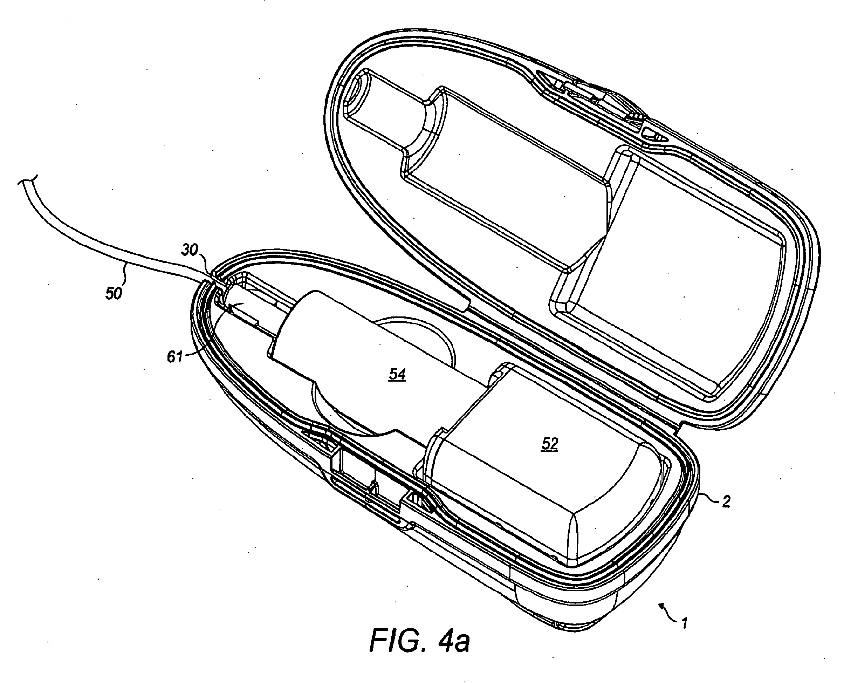

Turning now to figures 4a-c, these show three views of the case 1 with a pump and syringe held therein. An actuator pump 52 is shown received in the first section 8a of the recess 8 in the first portion 2 of the case 1. It can be seen that only part of the depth of the pump 52 is held in the recess 8, such that the remainder of the depth of the pump 52 can be accommodated by the first section 32a of the recess 32 in the second half 4 of the case 1.

A syringe 54 is shown received in the second section 8a of the recess 8. Again, only part of the depth of the syringe 54 is held in the recess 8, the remainder to be accommodated by the second section 32b of the recess 32 in the second half 4 of the case 1.

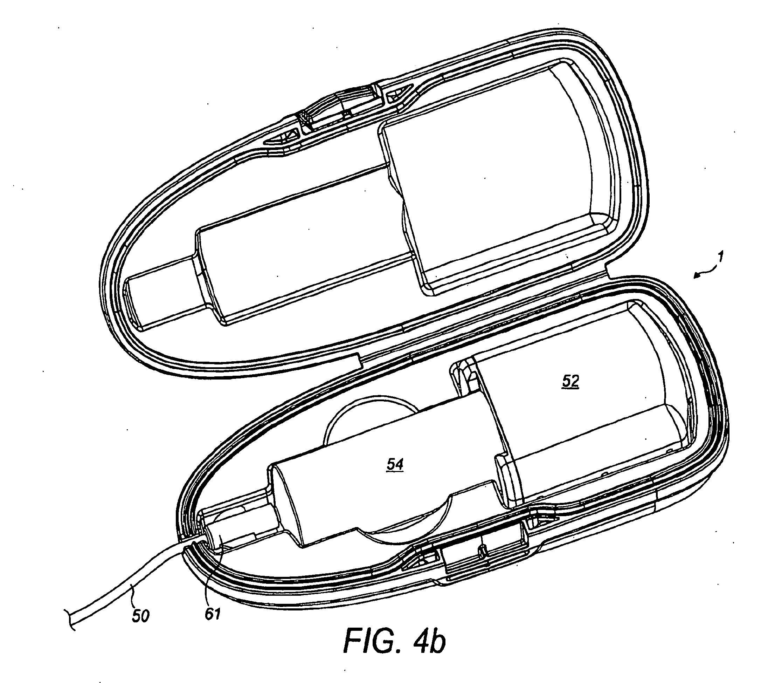

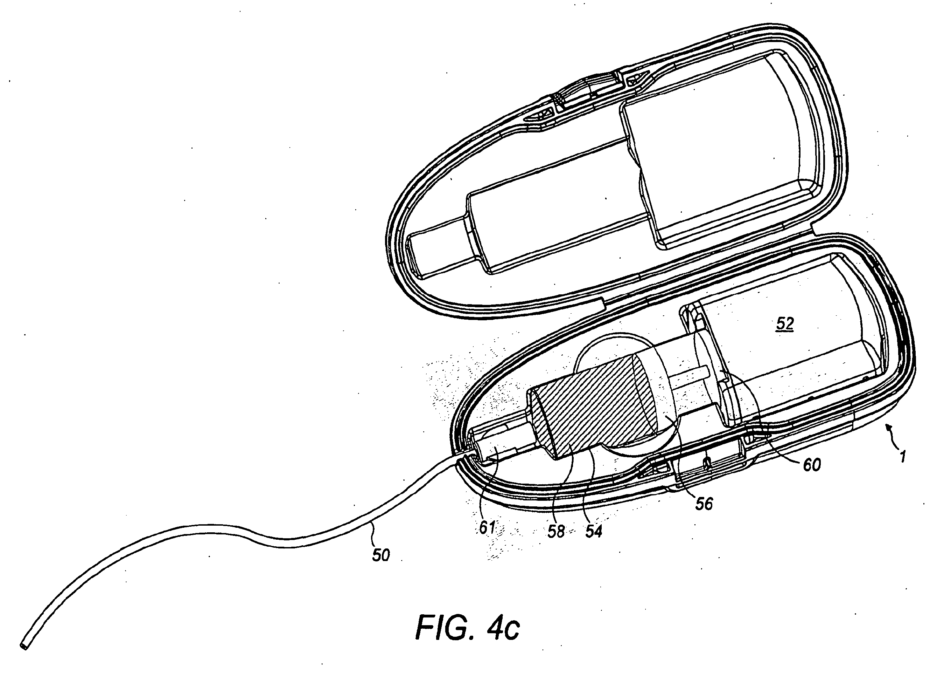

Whilst figures 4a and b show only the exterior of the pump 52 and the syringe 54, figure 4c shows some of the internal components. It can be seen that a piston 56 travels along the interior of the syringe 54, pushing a liquid medicine 58 into the cannula line 50. The piston 56 is in contact at its end distal from the liquid 58 with a plunger 60 of the pump 52. The pump 52 includes a power supply such as a power cell (battery) and a motor for progressively driving the plunger 60 (not shown).

It should be noted that the syringe 54 is a flangeless syringe. It is made of glass. The syringe 54 is not attached as such (e.g. by means of a slot) to the pump 52 but the two are merely abutting. In practice, it is not entirely necessary for the pump 52 and the syringe 54 even to abut each other as long as the syringe 54 can not move relative to the plunger 60. The case 1 achieves this requirement.

The pump 52 is designed without a narrow region to accommodate the plunger 60 of the type (labeled as 116) shown in the prior art in figure 8. Consequently the syringe 54 does not need to be pushed into engagement with the pump 52 as in the prior art. The case 1 and the internal cavity formed by the recess 8 in the first portion 2 of the case 1 are shaped and dimensioned to accommodate the pump 52 and the syringe 54 snugly and in mutual fixed relation. In this position, the pump 52 and the piston 56 of the syringe 54 are operably engaged. As previously mentioned, the ribs 12 in the recess 8 assist in holding the pump 52 securely in place. The recess 32 in the second portion 4 of the case 1 assists in retaining the pump 52 and the syringe 54 in place when the case 1 is shut.

The ability of the case 1 to hold the pump 52 and the syringe 54 as described above is advantageous because there is no requirement for the syringe 54 to be pushed into engagement with the pump 52. Rather, the case 1 holds the pump 52 and syringe 54 such that they can operate together. Consequently, a syringe made of a fragile material such as glass can be safely used. As a result, the liquid medicine 58 can be delivered to a patient as a pre-filled glass syringe so that there is no need for decanting the medicine 58 from one vessel to another. This is much safer for reasons explained above.

Another advantage of the case 1 is that once the pump 52 and the syringe 54 are in place and operating together, the case can be easily carried around on the person of a patient. This can be facilitated by a belt clip attached to the case or other similar means such as a neck or arm holster. As the case 1 has a rigid exterior it provides shock and impact protection for the pump-syringe unit. The complete envelopment of the pump 52 and the syringe 54 and the fact that they are stably supported around their entire extents assists in protecting them. Furthermore, as the case 1 is water resistant it protects the pump 52 and the syringe 54 from rain and other sources of liquid ingress.

In use, the cannula line 50 is inserted into a neck region 61 of the syringe 54 via a Luer container. The neck region 61 of the syringe 54 is narrower than the main body of the syringe 54 and is dimensioned to receive the cannula 50. The syringe 54 is then loosely attached to the pump 52 such that it fits over the plunger 60 of the pump 52. Then the combined unit is inserted into the case 1 such that the pump 52 is held in the first section 8a of the recess 8 in the first half 2 of the case 1 and the syringe 54 is held in the second section 8b of the recess 8. This is done simply by pushing the pump 52 and the syringe 54 into the recess 8, assisted by the finger recesses 28. There is no need to push the syringe 54 towards the pump 52 and hence the risk of shattering the syringe 54 is mitigated. At this stage, the piston 56 is disposed towards the end of the syringe 54 at which it was filled, distal from the end into which the cannula line 50 is to be inserted and near to the plunger 60 of the pump 52. Finally the cannula line 50 is pushed into the slot 30 so that it will not be damaged when the case 1 is shut. As mentioned previously, the pump 52 and the syringe 54 can be closely adjacent one another within a suitable tolerance but do not necessarily have to touch once inserted in the case 1.

The pump 52 is programmable to deliver the medicine 58 to the patient at an appropriate rate. Once the pump has been set running, the case 1 is closed and the clasp 5 is fastened. The plunger 60 of the pump 52 progressively pushes the piston 56 along the syringe, thereby pushing the medicine 58 out of the syringe through the cannula line.

When all the medicine 58 has been dispensed, the clasp 5 can be unfastened to re-open the case 1. The syringe 52 can then be removed. The removal is assisted by the presence of the finger recesses 58. A replacement pre-filled syringe can then be inserted into the case 1.

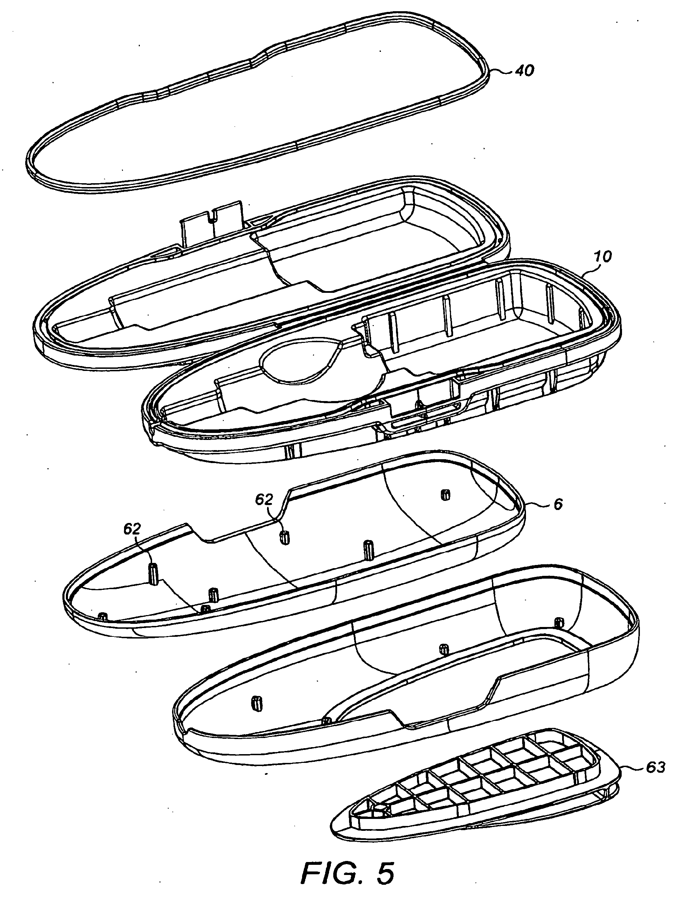

Figure 5 shows an exploded view of the case 1 in an open configuration shown from a similar angle to previous figures. The outer layer 6, the inner layer 10 and the rubber seal 40 are shown separately. The outer layer 6 has a number of dowels 62 which assist in locating the inner layer 10 correctly and holding the two together. This figure shows an extra raised section 63 on the outer shell of the case 1 which can be used for accommodating a manufacturer's logo or other design.

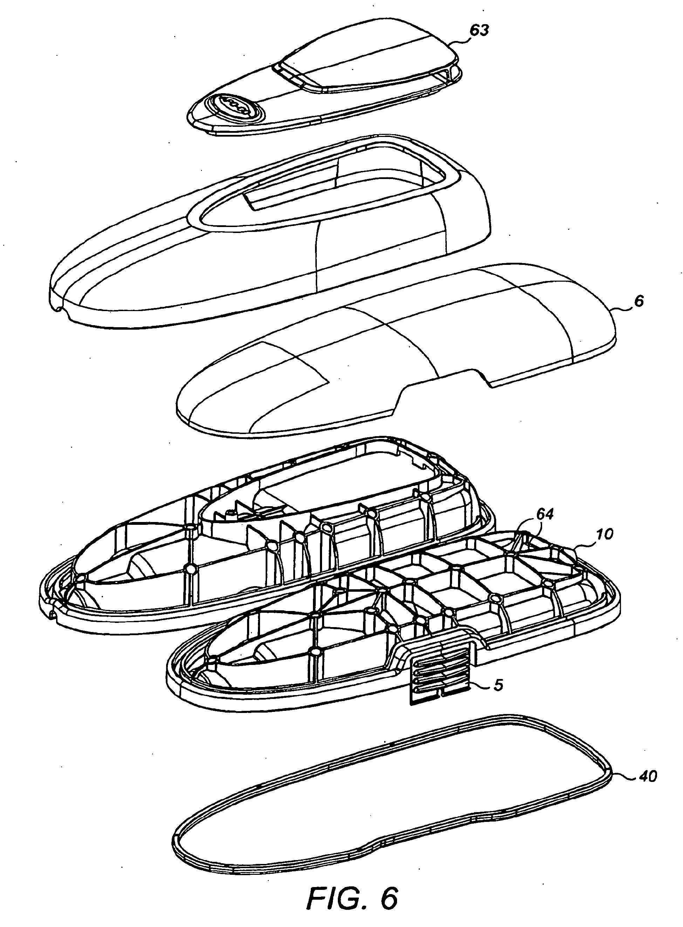

Figure 6 also shows an exploded view of the case 1 but showing the underside of the case as compared to previous figures. This figure also shows the raised section 63. It can be seen that the inner layer 10 comprises a network of ribs 64 arranged as a plurality of squares. As previously mentioned, the inner layer 10 is made of polypropylene which is naturally flexible to provide shock absorbance for the pump 52 and the syringe 54 and to enable multiple operations of the clasp 5 over the life of the case 1. These ribs 64 stiffen the inner layer 10 so that its structure is retained.

It will be appreciated that various details of the case 1 can be varied in other embodiments. The case 1 could be of a different external shape. The clasp 5 could be placed in different locations on the first and second portions 2,4 of the case 1 and could be placed vice versa on the opposite halves of the case 1. The hinge 3 could be of a different length or in a different position around the join of the two halves of the case 1. The recess 40a and the ridge 40b could be in the opposite halves of the case to their locations in the described embodiment.

An alternative fastening means could be used to close the case 1, such as a magnetic fastener or a tamper-evident locking mechanism. Thus the clasp 5 does not need to be formed as part of the inner layer 10 of the case 1 but could be formed separately. It is not necessary to use a hinge to join the two halves of the case 1 together but instead other fastening means could be used in multiple locations around the case such as clips.

The ribs 12 in the recess 8 in the first portion 2 of the case could differ in number and shape and size. Ribs or other formations could be provided in the second portion 4 of the case.

The notches 30 do not have to be cut in only one half of the case 1 but could be cut to lie in both halves.

The finger recesses 28 could be omitted and other means such as a releasable tab could be used to assist in insertion and removal of the syringe 54 in the case 1.

The particular materials of the various parts of the case 1 in the above embodiment are not essential and could be varied. For example, the outer portion 6 of the case 1 could be metal for aesthetic purposes. The rubber seal 40 could be made of an alternative material such as silicone.

The depth and dimensions of the syringe 54 and the pump 52 accommodated in each portion 2, 4 of the case 1 could be varied from that shown in the figures.

Although particularly advantageous for the type of syringe and pump described, the case 1 could be used to hold other types of syringe and pump, whether attached to each other or not. If desired an insert such as a plastic ring could be used between the pump and the syringe to provide extra protection against shattering of the syringe. Further, the shape of the recesses could be altered to accommodate attachment means for the pump for attaching to a user, such as lanyard dips for use with a neck cord. The corresponding dimensions of the sections 8 and 32 in the case 1 for holding the pump 52 and syringe 54 could be varied to accommodate different pumps and syringes and any inserts or attachment means used.

The case 1 could also be used with infusion equipment comprising a vessel other than a syringe, such as a cartridge, an ampoule or a carpoule.

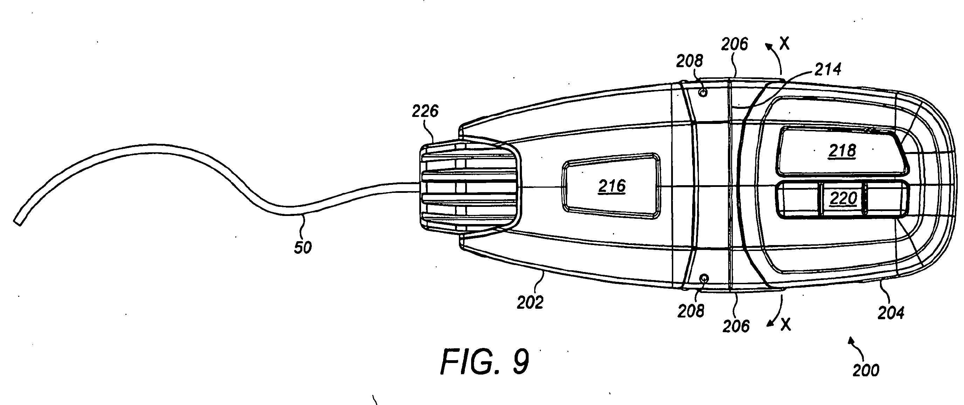

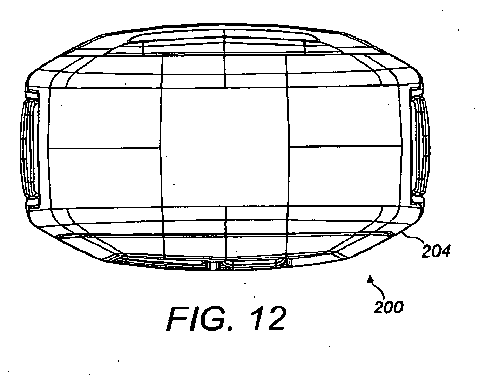

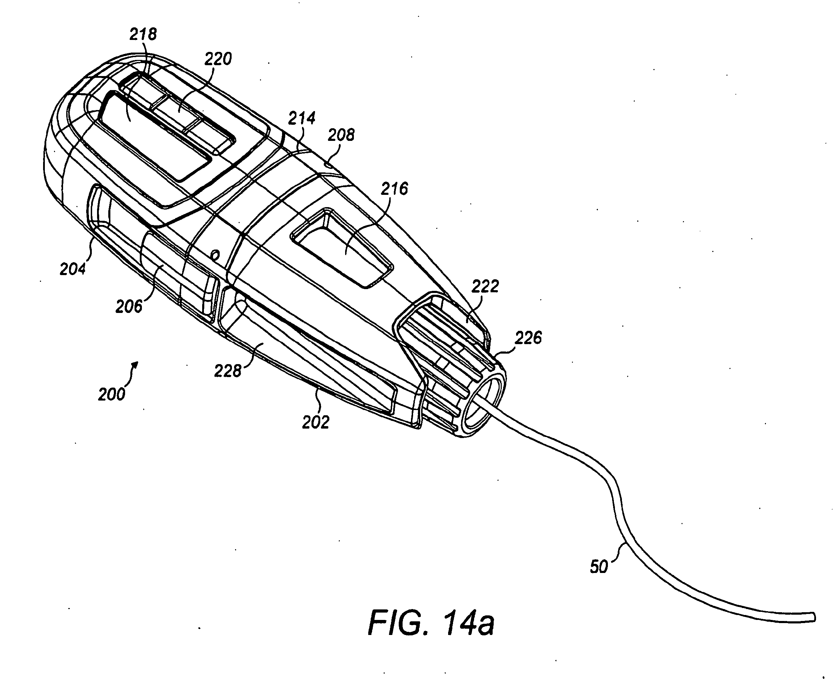

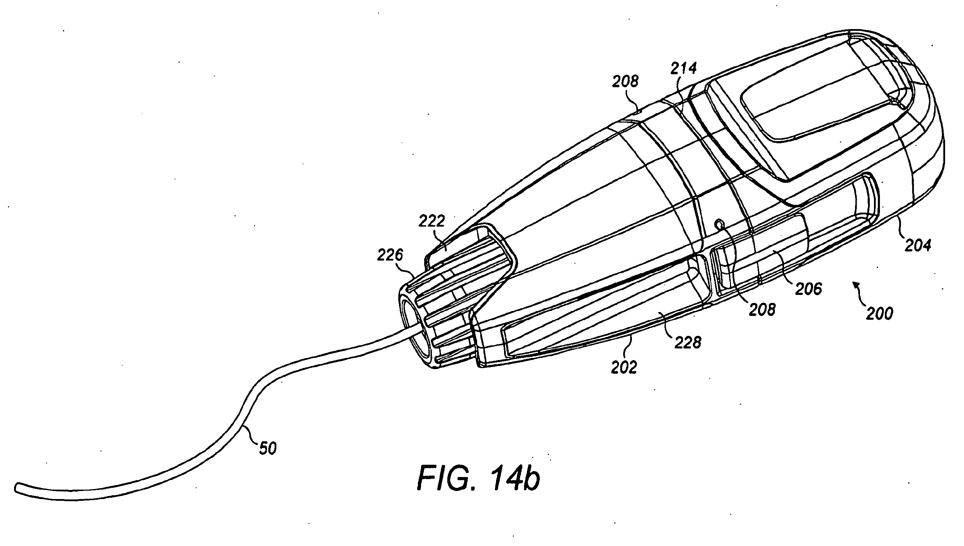

Referring to figures 9 to 15, there is shown a case 200 in accordance with a second embodiment of the invention. The case 200 comprises a front portion 202 and a rear portion 204. In general terms, the front portion is designed to accommodate a syringe and the rear portion is designed to accommodate a pump.

As with the case 1, the front and rear portions 202,204 are generally cuboid but rounded in shape, having a length greater than a width, the width being greater than a depth. Each is generally hollow and open at one of its smallest faces. The widths and the depths of the open face of each portion 202,204 are sized and shaped to match such that the first 202 and second 204 portions are able to be fitted together at their open faces to form an enclosure. When fitted together, the case 200 itself has a generally cuboid, rounded shape, having a length greater than a width, which in turn is greater than a depth.

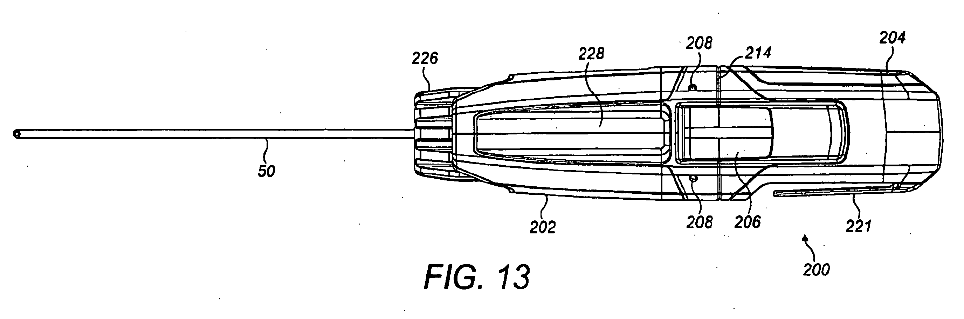

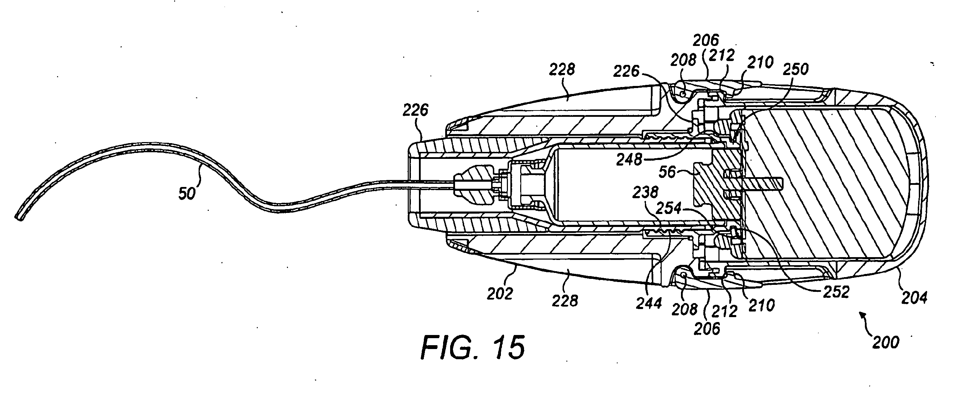

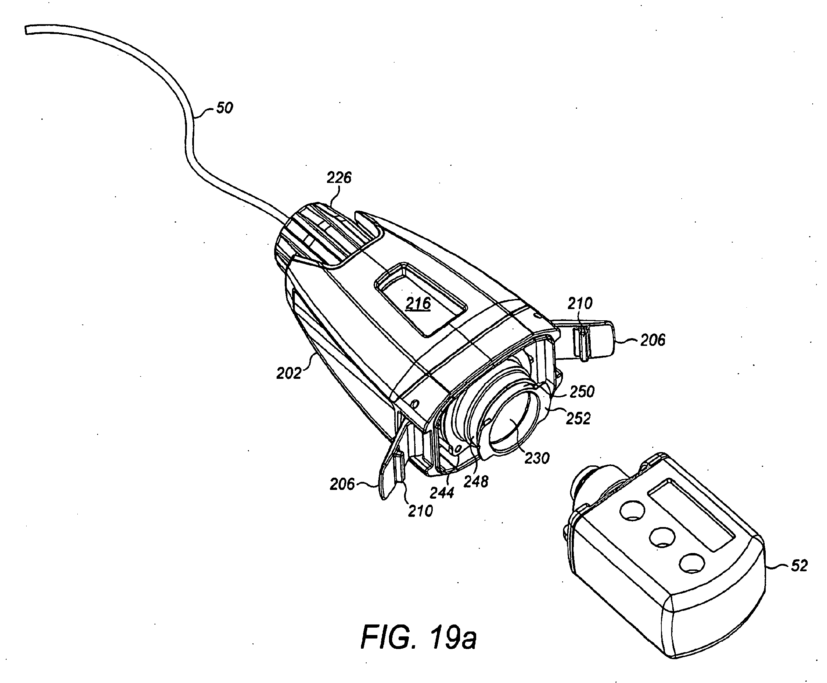

The front and rear portions 202, 204 are held removeably together by two clasps 206. The two clasps 206 take the form of generally planar wings hinged to the front portion 202 at hinges 208. The hinges 208 hold the wings towards the top of the case and the bottom of the case, such that the wings are disposed on the shortest edge (which defines the depth) of the front portion 202 (as viewed square on in figure 13 and termed herein the side of the front portion 202 and case 200). The wings are hinged to move in an arc which is in the same plane as the top and bottom of the case 200. This rotation is indicted by arrows X in figure 9. The clasps 206 have protrusions 210 on their inward-facing faces which snap-fit into corresponding detents 212 on the rear portion 204. These protrusions and detents can best be viewed in figures 15 and 19a. The joining line of the two halves of the case 200 is labeled as 214 in the figures.

The case 200 could be made of various materials. Suitable materials include a rigid thermoplastic such as acrylonitrile butadiene styrene (ABS), nylon, or a combination of both in various regions as appropriate. The case can be made from or covered in impact-dampening material to protect against shock damage if it should be dropped. However, the material could also be chosen for aesthetic purposes as well as or rather than functional considerations. Thus it could be made from various plastics or metals.

On the top side of the first portion 202, there is a window 216 cut out through which a user can view the amount of medicament remaining in a syringe. On the top side of the rear portion, there are two cut-outs. A first cut-out 218 is for viewing a screen of a pump. Such a screen shows the operational status of the pump. A second cut-out 220 is to enable the user to operate buttons of a pump. All these windows are shown to be squareish in shape and disposed generally in the centre of their respective portions of the case 200 but it will be appreciated that the number, shape and location of these windows could be varied to suit different syringes and pumps. It will also be appreciated that the windows could be filled with rigid or flexible transparent material if desired.

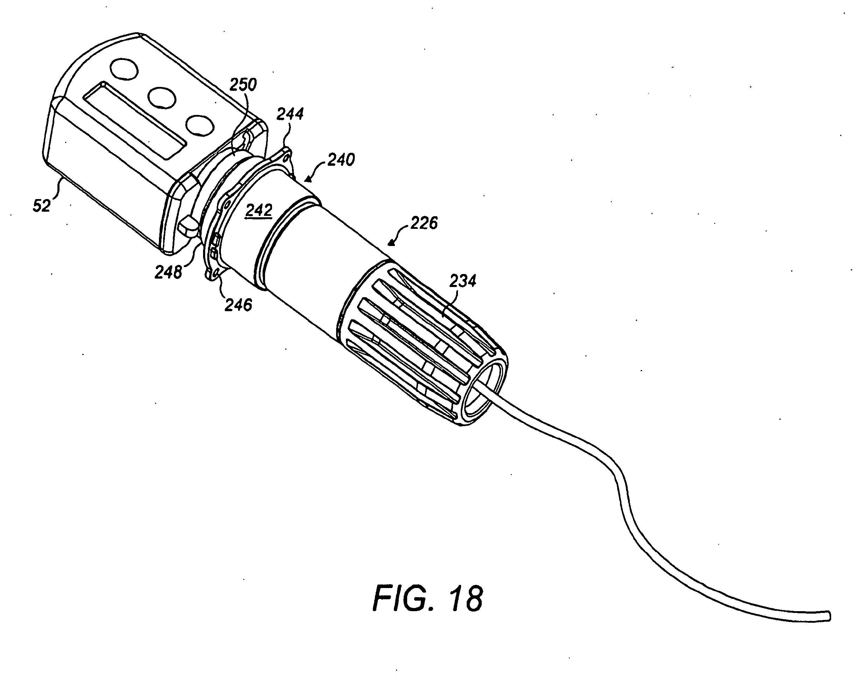

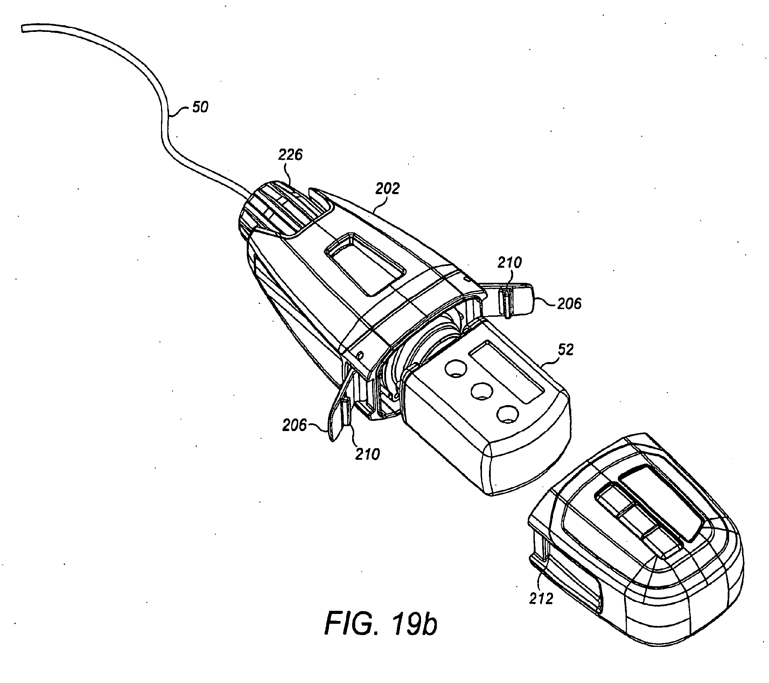

The screen and buttons can be seen on the pump 52 in figures 18 and 19. It should be noted that these details were omitted from previous figures.

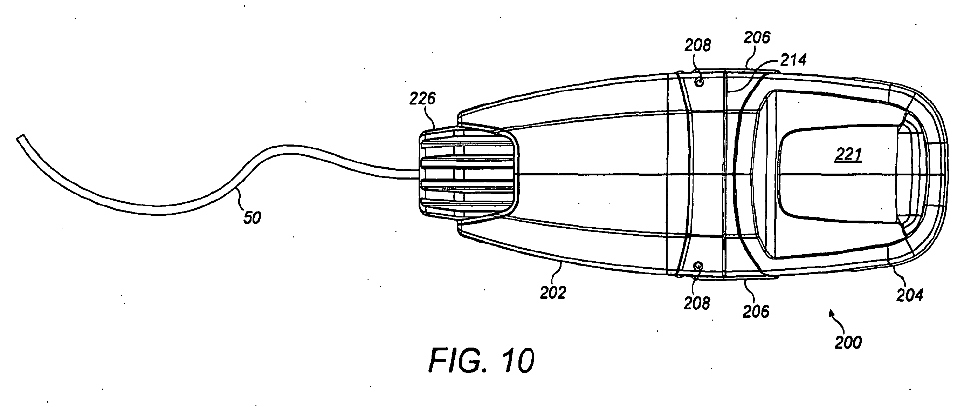

As seen in figures 10 and 13, the underside of the case 200 can include a belt clip 221.

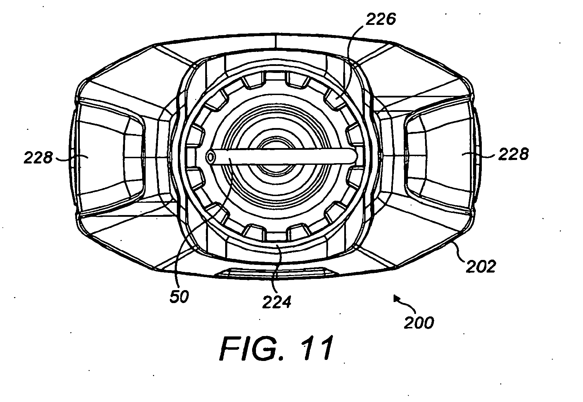

The end of the front portion 202 of the case 200 distal from the end which fits to the rear portion 204 is cut out into a squareish U-shape 222, having a depth through the side of the case. Thus the bottom of the U-shape is perpendicular to the length of the case and has a circular opening 224 (best viewed in figure 11). Out of this circular opening 224 can be seen protruding a syringe holder 226, to be described in more detail below. In the figures, a syringe is being held by the holder 226 so a cannula line 50 is exiting the holder 226 and hence the case 200.

It can be seen in figures 11 and 13-15 that the front portion 202 also has a cut-out portion 228 in each of its sides. However, this cut-out portion does not form a window but rather a recess, the inner face of which forms part of an inner extent of the front portion 202 in which the holder 226 can be held.

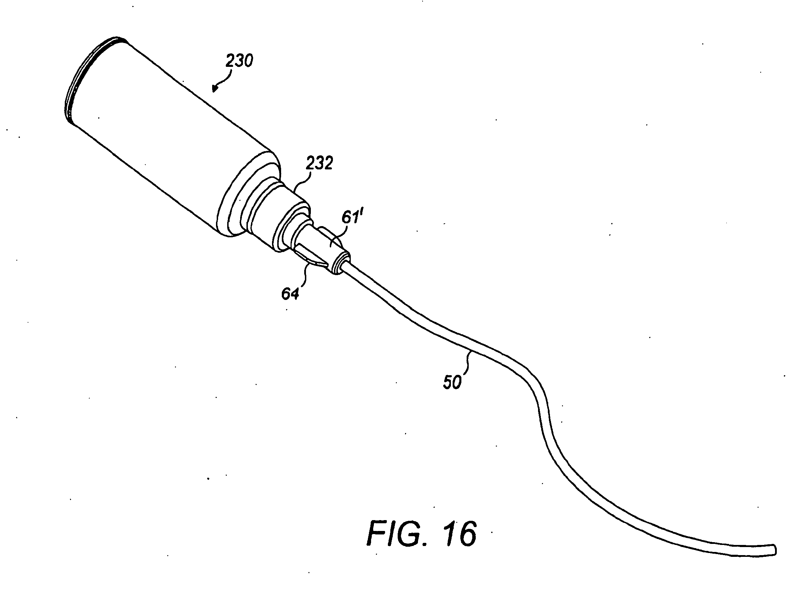

Figure 16 shows an example of a flangeless syringe 230 which can be held by the syringe holder 226. It is very similar to the syringe 54, although it has an extra intermediate portion 232 having a diameter inbetween that of the main part of the syringe and the neck portion 61'. The neck portion 61' bears a standard luer connector 64 for attachment of the cannula line 50. It will be appreciated that either syringe 50 or 230 could be accommodated by either type of case 1 or 200, by minor modifications to dimensions of parts of the case as necessary.

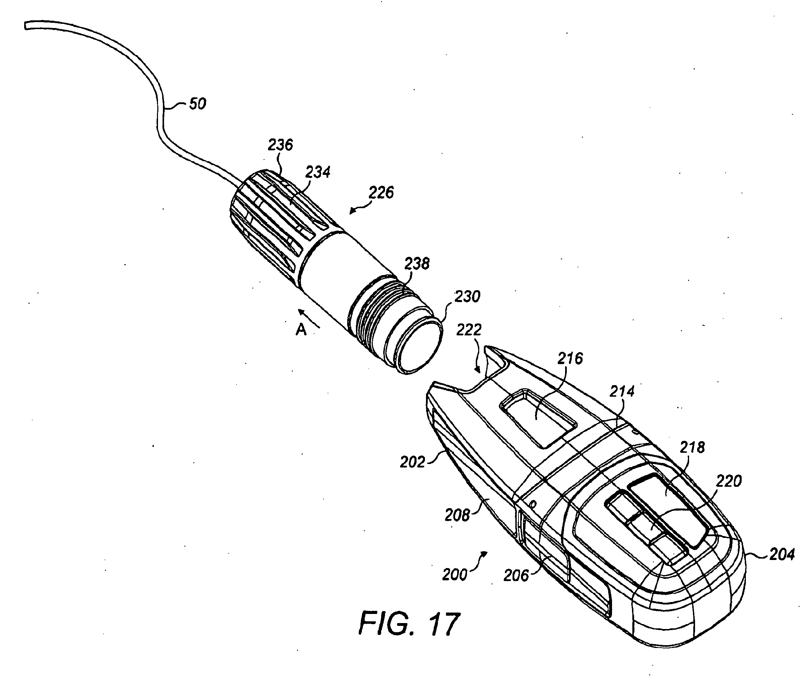

Turning now to figure 17, the syringe holder 226 can be seen in perspective view. The syringe holder 226 is generally cylindrical in shape and it is hollow, to thereby define a cavity for accommodating a syringe 230. A syringe 230 is inserted into the holder 226 in the direction of arrow A, from the end of the holder 226 which will be proximal to a pump 52 towards the distal end.

A portion of the distal end of the holder 226 has a user-operable means 234 in the form of ridges on its surface. When a syringe 230 is inserted into the holder 226, the cannula line 50 is inserted first and projects out of the distal end. The very distal end is a tapered region 236, which thereby limits movement of the syringe 230 into the holder 226 during insertion, so that the syringe protrudes from the proximal end of the holder 226 by about 0.5cm. It will be appreciated that the relative lengths of the tapered section, the user-operable means and the holder can be varied. Once so inserted, the syringe 230 is held snugly in the syringe holder 226.

A portion of the holder 226 towards the proximal end comprises a threaded portion 238. This is for attaching the holder to a pump attachment and thereby to the front portion 202 of the case, as will be described below.

Figure 17 shows the holder 226 in position to be inserted into the front portion 202 of the case. It is inserted into the cut-out distal end of the front portion 202, and specifically through the circular opening 224 within the cut-out U-shape 222. It will be understood that the two portions 202, 204 of the case do not need to be attached together when the holder 226 is inserted into the front portion 202. Furthermore, the syringe 230 could be inserted after the holder 226 is inserted into the front portion 202, although this is not preferred as it is easier to insert it into the free holder. The ability of the holder 226 to be removed from the case with a syringe is a useful feature of the embodiment because it allows a user to change for a new syringe without having to take the case 200 apart.

Figure 18 shows the holder 226 attached to a pump attachment 240. The pump attachment 240 comprises a first hollow, cylindrical portion 242 and a radial flange 244. The cylindrical portion 242 is dimensioned to engage with the threaded portion 238 of the holder 226 and has a corresponding screw thread 244 (visible in figure 15) on its inner surface for this purpose. The radial flange is at the end of the first cylindrical portion 242 proximal to where the pump attachment 240 will engage with a pump 52. The flange 244 comprises four screw holes 246 (three of which are visible in figure 18) evenly spaced around the flange for attachment to the front portion 202 of the case 200. Thus the pump attachment 240 is attached to the front portion 202 at the open end of the front portion 202, as can be seen in figure 19a.

For use, the pump attachment 240 is fitted onto the front portion 202 of the case 200 by inserting the cylindrical portion 242 into the front portion 202 from the open end of the front portion 202. Thus the front portion 202 comprises internal features to accommodate the cylindrical portion 242. In this embodiment the internal features which are used for this purpose comprise an internal wall which lies perpendicular to the direction of insertion of the pump attachment 240 into the front portion 202. However, it will be appreciated that other internal formations could be provided as appropriate depending on the design of attachment used - for example, the flange 244 could have some portions at different angles and the internal formations could be provided to receive screws at the appropriate angle. The flange 244 limits movement of the pump attachment 240 into the front portion 202 by butting up against the internal wall of the front portion 202, into which it can then be screwed. In this embodiment, it is intended to have the pump attachment 240 permanently attached to the front portion 244, although it will be appreciated that this need not be the case and that other attachment means could be used.

On the other side of the flange 244 to the cylindrical portion 242, the pump attachment 240 has a further cylindrical portion 248 dimensioned to fit snugly around the portion of a syringe 230 which projects beyond the end of the holder 226 (as described with reference to figure 17). Beyond this is a yet further, smaller diameter cylindrical portion 250, dimensioned to fit snugly around the part of a pump 52 from which a plunger 60 exits. The further cylindrical portion 248 has an axial length (i.e. along the length of the case 200) such that the step between it and the yet further cylindrical portion 250 is beyond the projecting syringe 230 to thereby avoid contact damage to the syringe 230. This can be seen most clearly in figure 15. This gap can be filled with a compression seal to further protect the syringe 230. Thus, as explained with respect to the first embodiment, there is no need for the syringe 230 to be in actual contact with the pump as long as they are held in mutual fixed relation.

The end of the yet further cylindrical portion distal from the screw-threaded cylindrical portion 242 of the pump attachment 240 i.e. at the pump attachment end, has a dual-winged flange 252, as shown in figure 19a. This flange is not visible in figure 18 because it has been engaged with a corresponding recess on the pump 52. In this embodiment, the engagement is effected by rotating and pushing the pump onto the pump attachment 240.

It can be seen in figure 15 that in this embodiment, the wings of the flange 252 comprise two portions of different radii, forming a "step" 254. This detail is included for engagement with a particular type of pump and it will be appreciated that the shape and radius of the flange 252 can be varied in dependence on the type of pump being used.

In use, as previously mentioned, the syringe holder 226 can be inserted into and removed from the pump attachment 240 by engagement or disengagement of the respective screw threads 238 and 244. In view of the fact that the pump attachment 240 Is fixed to the front portion 202 of the case 200, this in effect allows the syringe holder 226 with (or without) a syringe 230 in place to be inserted into and removed from the case 200. Preferably when removed, a syringe 230 can be replaced. Figure 19b shows that, having inserted a "loaded" syringe holder 226, a pump 52 can then be engaged with the pump attachment 240 by twisting and pushing, as previously mentioned. The rear portion 204 of the case 200 can then be pushed over the pump 52 and the two portions of the case 200 attached together by means of the clasps 206. Alternatively, the pump 52 can be placed in the rear portion 204 of the case 200 first and then engaged with the pump attachment 240. The two portions of the case 200 do not need to be unattached for removal of the syringe holder 226 because the pump can remain engaged with the pump attachment 240 whilst the syringe holder 226 is unscrewed from the pump attachment 240 and the syringe 230 is replaced.

It will be understood by the skilled person that in mechanical terms, in this embodiment, the front and rear portions 202, 204 of the case 200 are not needed to hold the pump and syringe in operable engagement, but rather, it is the pump attachment 240 and the syringe holder 226 which do the mechanical work. The engagement of the syringe holder 226 to the pump attachment 240 (in this embodiment by means of the screw thread 238 on the syringe holder 226 and the corresponding screw thread 244 on the pump attachment 240) provides a resistance to movement of the plunger 60 of the pump 52 to prevent the syringe 230 being pushed out of engagement with the pump 52. Thus the syringe 230 and pump 52 are held securely in operable engagement. Furthermore, because the resistive force is provided by interaction of the pump attachment 240 (which is engaged with the pump) and the syringe holder 226, the syringe 230 does not have to bear the force of movement of the piston 56. Thus damage to the syringe, if formed of a brittle material such as glass, is mitigated. The envelopment of the syringe 230 by the syringe holder 226 and the pump attachment 240 also provides impact protection to the syringe 230.

The case 200 is nevertheless useful as it provides protection for the pump 52, the syringe 230 and syringe holder 226 and can be provided with seals around the syringe holder 226 and at the join 214 between its two halves, so as to provide protection against water ingress as well as impact protection, portability and a convenient means of attachment to a user, for example on a belt. It provides stable support of a pump and syringe, although this is achieved with assistance from the syringe holder 226.

It will further be appreciated that in view of the secure attachment of the pump attachment 240 to the front portion 202 of the case, then it is in effect the front portion 202 of the case 200 as a whole providing the mechanical resistance together with the syringe holder 226. This would also be the case in an alternative embodiment in which the pump attachment 240 is integral with the front portion 202 of the case. However, it can be understood that the mechanical effect can be achieved by the provision merely of a pump attachment 240 of the type shown which co-operates with the syringe holder 226.

Various alternatives to features of the case 200 can be envisaged. The relative lengths of the syringe holder 226 and the pump attachment 240 could be varied. The pump attachment could be significantly longer so as to accommodate the majority of a syringe, the holder 226 acting as a stop to prevent movement of a syringe away from a pump. Thus the tapered feature of the holder 226 is not essential. The length of protrusion of a syringe out of the holder could be varied independently of variations in the relative lengths of the holder and pump attachment.

The syringe holder 226 could be engaged with the pump attachment 240 by different means than a screw thread, such as a push-fit or clips or other mechanical means such as a detent mechanism. The pump attachment 240 could be attached to the front portion 202 of the case by means other than screws or, as mentioned above, could be integrally formed therewith. The user-operable means 234 on the syringe could take a different form than ribs, or could be formed from a different, higher friction material such as rubber.

All the shapes and dimensions shown in the figures could be varied, and in particular the stepped portions shown on the pump attachment 240 could be varied. The details of the case 200 could be varied to enable it to be used with different types of syringes and pumps. Extra rubber buffers could be used in the case for extra impact protection. The two halves of the case do not necessarily need to be of substantially equal size and could be joined at different faces and by different means such as a different type of clip or a push-fit.

The case 200 could also be used with infusion equipment comprising a vessel other than a syringe, such as a cartridge, an ampoule or a carpoule.

## Claims

1. An infusion apparatus comprising a vessel (230) having a tubular bore and a piston; a separate actuator pump (52) for the vessel (230); a holder (226) shaped and dimensioned to hold the vessel (230); and
a pump attachment (240) with which the holder (226) can be engaged; wherein the pump attachment (240) is shaped and dimensioned to be removeably engagable with the actuator pump (52), to thereby, when the holder (226) and the pump attachment (240) are so engaged, removeably hold the actuator pump (52) and the vessel (230) in mutual fixed relation such that the actuator pump (52) is operably engaged with the piston (56) of the vessel (230) without attaching the vessel (230) to the actuator pump (52).

2. An apparatus according to claim 1, wherein the pump attachment (240) and the holder (226) comprise corresponding engageable means (252) for allowing the said engagement and wherein the engageable means can be engaged for securely fitting the holder (226) to the pump attachment (240).

3. An apparatus according to claim 1, wherein the pump attachment (240) is shaped and dimensioned to be removeably engageable with the actuator pump (52) by means of one or more wings on one of the pump attachment (240) and the actuator pump (52) and corresponding one or more recesses on the other of the pump attachment (240) and the actuator pump (52) which wings and recesses interengage.

4. An apparatus according to claim 3, wherein the wings comprise a stepped radius arranged to engage with a corresponding stepped recess.

5. An apparatus according to claim 1, wherein, when engaged, the holder (226) and the pump attachment (240) define a tubular bore into which the vessel (230) can fit snugly.

6. An apparatus according to claim 5, wherein the tubular bore is arranged to cover a substantial part of the vessel (230).

7. An apparatus according to claim 5 or claim 6, wherein a substantial part of the tubular bore is formed by the holder (226).

8. An apparatus according to any of claims 5 to 7, wherein, when engaged, the holder (226) and the pump attachment (240) are arranged to limit movement of the vessel (230) out of the bore towards an end distal from the pump attachment (240).

9. An apparatus according to claim 8, wherein the movement limitation is effected by tapering (236) of the bore.

10. An apparatus according to claim 8 or claim 9, wherein the movement of the vessel (230) is limited such that, when the vessel (230) is held in the holder (226), an open end of the vessel (230) is enveloped by the bore to thereby protect it.

11. An apparatus according to any preceding claim, wherein the holder (226) comprises user-operable means for assisting engagement with and disengagement from the pump attachment (240).

12. An apparatus according to any preceding claim, further comprising a case (200) defining an internal cavity that is shaped and dimensioned to accommodate the holder (226) and pump attachment (240) when fitted together and holding the vessel (230) removeably engaged with the accuator pump (52).

13. A kit comprising:
a pre-filled vessel (230) having a tubular bore and a piston (56);
a seperate actuator pump (52) for the pre-filled vessel (230) ;
a holder (226) for holding the vessel (230); and
a pump attachment (240) with which the holder (226) can engage; wherein the pump attachment (240) is shaped and dimensioned to be removeably engagable with the actuator pump (52), to thereby, when the holder (226) is engaged with the pump attachment (240), removeably hold the actuator pump (52) and the vessel (230) in mutual fixed relation such that the actuator pump (52) is operably engaged with the piston (56) of the vessel (230) without attaching the vessel (230) to the actuator pump (52).

14. A method of holding an infusion apparatus, comprising a vessel (230) having a tubular bore and a separate actuator pump (52) for the vessel (230), comprising inserting the pre-filled vessel (230) into a holder (226); engaging the holder (226) with a pump attachment (240); and
removeably engaging the pump attachment (240) with an actuator pump (52) to thereby hold the pump (52) and vessel (230) in mutual fixed relation such that the actuator pump (52) is operably engaged with a piston (56) of the vessel (230) without attaching the vessel (230) to the actuator pump (52).

15. A portable case for an infusion apparatus comprising a pre-filled vessel (230) having a tubular bore, a piston (56) of the vessel (230) and a separate actuator pump (52) for the vessel (230), said case having a shell-like body defining an internal cavity that is shaped and dimensioned to accommodate said actuator pump (52) and the vessel (230), said case being shaped within the cavity for removably holding the actuator pump (52) and the vessel (230) in mutual fixed relation such that the actuator pump (52) is operably engaged with the piston (56) of the vessel (230) without attaching the vessel (230) to the actuator pump.

## Patentansprüche

1. Infusionsgerät mit einem Gefäß (230), das eine rohrförmige Bohrung und einen Kolben umfasst, einer getrennten Aktorpumpe (52) für das Gefäß (230); einem Halter (226), der geformt und bemessen ist, um das Gefäß (230) zu halten; einem Pumpenanschluss (240), mit dem der Halter (226) in Eingriff bringbar ist, wobei der Anschluss (240) geformt und bemessen ist, um mit der Aktorpumpe (52) in lösbaren Eingriff bringbar zu sein, um dadurch, wenn der Halter (226) und der Pumpenanschluss (240) so im Eingriff sind, die Aktorpumpe (52) und das Gefäß (230) in einer solchen festen Beziehung zueinander zu halten, dass die Aktorpumpe (52) in Funktionseingriff mit dem Kolben (56) des Gefäßes (230) steht, ohne das Gefäß (230) an der Aktorpumpe (52) zu befestigen.

2. Infusionsgerät nach Anspruch 1, bei dem der Pumpenanschluss (240) und der Halter (226) einander entsprechende Eingriffsmittel (252) umfassen, um den Eingriff zu ermöglichen, und bei dem die Eingriffsmittel in Eingriff bringbar sind, um den Halter (226) sicher an den Pumpenanschluss (240) anzufügen.

3. Infusionsgerät nach Anspruch 1, bei dem der Pumpenanschluss (240) geformt und bemessen ist, um mit der Aktorpumpe (52) durch einen oder mehrere Flügel, die an einem unter dem Pumpenanschluss (240) und der Aktorpumpe (52) ausgewählten Teil vorgesehen sind, und entsprechende eine oder mehrere Aussparungen, die an dem anderen unter dem Pumpenanschluss (240) und der Aktorpumpe (52) ausgewählten Teil vorgesehen sind, in lösbaren Eingriff bringbar zu sein, wobei die Flügel und die Aussparungen zusammenwirken.

4. Infusionsgerät nach Anspruch 3, bei dem die Flügel eine gestufte Strebe umfassen, die eingerichtet ist, um in eine entsprechende gestufte Vertiefung einzugreifen.

5. Infusionsgerät nach Anspruch 3, bei dem im Eingriff der Halter (226) und der Pumpenanschluss (240) eine rohrförmige Bohrung definieren, in die das Gefäß (230) genau einpassbar ist.

6. Infusionsgerät nach Anspruch 5, bei dem die rohrförmige Bohrung eingerichtet ist, einen wesentlichen Teil des Gefäßes (230) zu überdecken.

7. Infusionsgerät nach Anspruch 5 oder Anspruch 6, bei dem ein wesentlicher Teil der rohrförmigen Bohrung von dem Halter (226) gebildet ist.

8. Infusionsgerät nach einem der Ansprüche 5 bis 7, bei dem der Halter (226) und der Pumpenanschluss (240) im Eingriff miteinander eingerichtet sind, eine Bewegung des Gefäßes (230) aus der Bohrung heraus in Richtung eines vom Pumpenanschluss (240) abgewandten Endes zu begrenzen.

9. Infusionsgerät nach Anspruch 8, bei dem die Begrenzung der Bewegung durch eine Verjüngung (236) der Bohrung erreicht ist.

10. Infusionsgerät nach Anspruch 8 oder Anspruch 9, bei dem die Bewegung des Gefäßes (230) so begrenzt ist, dass wenn das Gefäß (230) im Halter (226) gehalten ist, ein offenes Ende des Gefäßes (230) von der Bohrung umhüllt ist, um es dadurch zu schützen.

11. Infusionsgerät nach einem beliebigen der vorhergehenden Ansprüche, bei dem der Halter (226) benutzerbetätigbare Mittel zum Unterstützen und Lösen des Eingriffs mit dem Pumpenanschluss (240) umfasst.

12. Infusionsgerät nach einem beliebigen der vorhergehenden Ansprüche, ferner mit einem Gehäuse (200), das einen internen Hohlraum definiert, der geformt und bemessen ist, um den Halter (226) und den Pumpenanschluss (240) in zusammengefügtem Zustand aufzunehmen und das Gefäß (230) in lösbarem Eingriff mit der Aktorpumpe (52) zu halten.

13. Teilesatz mit:
einem vorbefüllten Gefäß (230), das eine rohrförmige Bohrung aufweist;
einer Aktorpumpe (52) für das vorbefüllte Gefäß (230);
einem Halter (226) zum Halten des Gefäßes (230); und
einem Pumpenanschluss (240), mit dem der Halter (226) in Eingriff bringbar ist, wobei der Pumpenanschluss (240) geformt und bemessen ist, um mit der Aktorpumpe (52) in lösbaren Eingriff bringbar zu sein, um dadurch, wenn der Halter (226) im Eingriff mit dem Pumpenanschluss (240) ist, die Pumpe (52) und das Gefäß (230) in einer lösbar festen Beziehung zueinander so zu halten, dass die Aktorpumpe (52) in Funktionseingriff mit einem Kolben (56) des Gefäßes (230) ist, ohne das Gefäß (230) an der Aktorpumpe (52) zu befestigen.

14. Verfahren zum Halten eines Infusionsgeräts mit einem Gefäß (230), das eine rohrförmige Bohrung aufweist, und einer getrennten Aktorpumpe (52) für das Gefäß (230), welches umfasst:
Einführen des vorbefüllten Gefäßes (230) in einen Halter (226);
In Eingriff Bringen des Halters (226) mit einem Pumpenanschluss (240); und
In lösbaren Eingriff Bringen des Pumpenanschlusses (240) mit einer Aktorpumpe (52), um dadurch die Pumpe (52) und das Gefäss (230) in einer festen Beziehung zueinander so zu halten, dass die Aktorpumpe (52) in Funktionseingriff mit einem Kolben (56) des Gefäßes (230) ist, ohne das Gefäß (230) an der Aktorpumpe (52) zu befestigen.

15. Tragbares Gehäuse für ein Infusionsgerät mit einem Gefäß (230), das eine rohrförmige Bohrung aufweist, und einer getrennten Aktorpumpe (52) für das Gefäß (230), wobei das Gehäuse einen schalenartigen Körper hat, der einen internen Hohlraum definiert, der geformt und bemessen ist, um die Aktorpumpe (52) und das Gefäß (230) aufzunehmen, wobei das Gehäuse in dem Hohlraum geformt und bemessen ist, um die Aktorpumpe (52) und das Gefäß (230) in lösbar festem Eingriff miteinander so zu halten, dass die Aktorpumpe (52) in Funktionseingriff mit einem Kolben (56) des Gefäßes (230) ist, ohne das Gefäß (230) an der Aktorpumpe zu befestigen.

## Revendications

1. Appareil de perfusion comprenant un récipient (230) possédant un alésage tubulaire et un piston ; une pompe à actionneur séparée (52) pour le récipient (230) ; un dispositif de retenue (226) façonné et dimensionné de façon à retenir le récipient (230) ; et
une fixation de pompe (240) avec laquelle le dispositif de retenue (226) peut être solidarisé ; la fixation de pompe (240) étant façonnée et dimensionnée de façon à être solidarisée de manière amovible avec la pompe à actionneur (52), ce qui permet par conséquent, lorsque le dispositif de retenue (226) et la fixation de pompe (240) sont solidarisés ainsi, de maintenir de manière amovible la pompe à actionneur (52) et le récipient (230) en relation fixe mutuelle de sorte que la pompe à actionneur (52) soit solidarisée de façon opérationnelle avec le piston (56) du récipient (230) sans attacher le récipient (230) à la pompe à actionneur (52).

2. Appareil selon la revendication 1, la fixation de pompe (240) et le dispositif de retenue (226) comprenant des moyens solidarisables correspondants (252) pour permettre ladite solidarisation, et les moyens solidarisables pouvant être solidarisés pour monter fermement le dispositif de retenue (226) sur la fixation de pompe (240).

3. Appareil selon la revendication 1, la fixation de pompe (240) étant façonnée et dimensionnée pour être solidarisable de manière amovible avec la pompe à actionneur (52) grâce à une ou plusieurs ailettes sur l'un des postes suivants, soit la fixation de pompe (240) soit la pompe à actionneur (52), et à un ou plusieurs évidements correspondants sur l'autre poste, soit la fixation de pompe (240) soit la pompe à actionneur (52), dont les ailettes et les évidements peuvent être solidarisés réciproquement.

4. Appareil selon la revendication 3, les ailettes comportant un rayon à gradins agencé de façon à se solidariser avec un évidement à gradins correspondant.

5. Appareil selon la revendication 1, lorsqu'ils sont en solidarisation, le dispositif de retenue (226) et la fixation de pompe (240) définissant un alésage tubulaire dans lequel le récipient (230) peut s'adapter étroitement.

6. Appareil selon la revendication 5, l'alésage tubulaire étant agencé de façon à couvrir une partie sensible du récipient (230).

7. Appareil selon la revendication 5 ou la revendication 6, une partie sensible de l'alésage tubulaire étant formée par le dispositif de retenue (226).

8. Appareil selon l'une quelconque des revendications 5 à 7, lorsqu'ils sont en solidarisation, le dispositif de retenue (226) et la fixation de pompe (240) étant agencés de façon à limiter le mouvement du récipient (230) hors de l'alésage vers une extrémité qui est distale par rapport à la fixation de pompe (240).

9. Appareil selon la revendication 8, la limitation du mouvement étant réalisée grâce à un amincissement (236) de l'alésage.

10. Appareil selon la revendication 8 ou la revendication 9, le mouvement du récipient (230) étant limité de sorte que, lorsque le récipient (230) est maintenu dans le dispositif de retenue (226), une extrémité ouverte du récipient (230) sera enveloppée par l'alésage afin d'assurer ainsi sa protection.

11. Appareil selon l'une quelconque des revendications précédentes, le dispositif de retenue (226) comprenant des moyens utilisables par un utilisateur pour faciliter la solidarisation avec la fixation de pompe (240) et la désolidarisation au niveau de celle-ci.

12. Appareil selon l'une quelconque des revendications précédentes, comprenant en outre un boîtier (200) définissant une cavité interne qui est façonnée et dimensionnée pour recevoir le dispositif de retenue (226) et la fixation de pompe (240), quand ils sont montés ensemble, et retenir le récipient (230) solidarisé de manière amovible avec la pompe à actionneur (52).

13. Kit comprenant :
un récipient pré-rempli (230) possédant un alésage tubulaire et un piston (56) ;
une pompe à actionneur séparée (52) pour le récipient pré-rempli (230) ;
un dispositif de retenue (226) pour maintenir le récipient (230) ; et
une fixation de pompe (240) avec laquelle le dispositif de retenue (226) peut se solidariser ; la fixation de pompe (240) étant façonnée et dimensionnée de façon à être solidarisée de manière amovible avec la pompe à actionneur (52), ce qui permet par conséquent, lorsque le dispositif de retenue (226) est solidarisé avec la fixation de pompe (240), de maintenir de manière amovible la pompe à actionneur (52) et le récipient (230) en relation fixe mutuelle de sorte que la pompe à actionneur (52) soit solidarisée de façon opérationnelle avec le piston (56) du récipient (230) sans attacher le récipient (230) à la pompe à actionneur (52).

14. Procédé permettant de retenir un appareil de perfusion, comprenant un récipient (230) possédant un alésage tubulaire et une pompe à actionneur séparée (52) pour le récipient (230), comprenant les opérations consistant à :
insérer le récipient pré-rempli (230) dans un dispositif de retenue (226) ;
solidariser le dispositif de retenue (226) avec une fixation de pompe (240) ; et
solidariser de manière amovible la fixation de pompe (240) avec une pompe à actionneur (52), ce qui permet par conséquent de maintenir la pompe (52) et le récipient (230) en relation fixe mutuelle de sorte que la pompe à actionneur (52) soit solidarisée de façon opérationnelle avec un piston (56) du récipient (230) sans attacher le récipient (230) à la pompe à actionneur (52).

15. Boîtier portatif pour un appareil de perfusion comprenant un récipient pré-rempli (230) possédant un alésage tubulaire, un piston (56) de récipient (230) et une pompe à actionneur séparée (52) pour le récipient (230), ledit boîtier ayant un corps de type carter qui définit une cavité interne laquelle est façonnée et dimensionnée de façon à recevoir ladite pompe à actionneur (52) et le récipient (230), ledit boîtier étant façonné à l'intérieur de la cavité pour maintenir de manière amovible la pompe à actionneur (52) et le récipient (230) en relation fixe mutuelle de sorte que la pompe à actionneur (52) soit solidarisée de façon opérationnelle avec le piston (56) du récipient (230) sans attacher le récipient (230) à la pompe à actionneur.
